# EUROPEAN PATENT SPECIFICATION

(11) **EP 1 394 537 B1**
(45) Date of publication and mention of the grant of the patent: **10.08.2011**
(21) Application number: 02733305.3
(22) Date of filing: 05.06.2002
(51) Int. Cl.: H01J 49/16, G01N 27/62, H01J 49/40, H01J 49/42

(54) **DEVICE AND METHOD FOR DETECTING TRACE AMOUNTS OF ORGANIC COMPONENTS**
VORRICHTUNG UND VERFAHREN ZUM NACHWEIS VON SPUREN ORGANISCHER BESTANDTEILE
DISPOSITIF ET PROCEDE DE DETECTION DE QUANTITES INFIMES DE COMPOSANTS ORGANIQUES

(30) Priority: 06.06.2001 JP 2001170753; 06.06.2001 JP 2001170778; 06.06.2001 JP 2001170784; 29.06.2001 JP 2001198574; 06.11.2001 JP 2001340487; 06.11.2001 JP 2001340495; 22.02.2002 JP 2002045801
(43) Date of publication of application: 03.03.2004
(60) Divisional application: 09165288.3
(73) Proprietor: MITSUBISHI HEAVY INDUSTRIES, LTD., Tokyo (JP)
(72) Inventor: DEGUCHI, Yoshihiro, c/o Mitsubishi Heavy Ind. Ltd, Nagasaki 851-0392 (JP); FUKUDA, Norihiro,c/o Mitsubishi Heavy Ind. Ltd, Nagasaki 851-0392 (JP); DOBASHI, Shinsaku,c/o Mitsubishi Heavy Ind. Ltd, Nagasaki 851-0392 (JP); HORI, Junichiro,c/o Mitsubishi Heavy Ind. Ltd, Nagasaki 851-0392 (JP); KUBOTA, Takahiro,c/o Mitsubishi Heavy Ind. Ltd, Nagasaki 851-0392 (JP); NODA, Matsuhei,c/o Mitsubishi Heavy Ind. Ltd, Nagasaki 851-0392 (JP); HIRAKI, Akio,c/o Mitsubishi Heavy Ind. Ltd, Nagasaki 851-0392 (JP)
(74) Representative: HOFFMANN EITLE
(86) International application number: PCT/JP2002/005527
(87) International publication number: WO 2002/101376

(56) References cited:
- EP-A1- 1 102 004
- JP-A- 9 243 601
- JP-A- 10 265 243
- JP-A- 11 224 645
- JP-A- 11 253 796
- JP-A- 11 344 477
- JP-A- 53 042 878
- JP-A- 2000 275 218
- JP-A- 2000 301 126
- JP-A- 2000 321 246
- JP-A- 2000 329 743
- JP-A- 2000 354 878
- JP-A- 2001 147 216
- US-B1- 6 204 500
- GREBNER T L ET AL: "Dissociation rates of energy-selected benzene cations: comparison of experimental results from ion cyclotron resonance and cylindrical ion trap time-of-flight mass spectrometry1" INTERNATIONAL JOURNAL OF MASS SPECTROMETRY, ELSEVIER SCIENCE PUBLISHERS, AMSTERDAM, NL, vol. 185-187, 29 April 1999 (1999-04-29), pages 517-532, XP004163698 ISSN: 1387-3806
- COCHRAN J W ET AL: "Recent developments in the high-resolution gas chromatography of polychlorinated biphenyls" 28 May 1999 (1999-05-28), JOURNAL OF CHROMATOGRAPHY A, ELSEVIER, AMSTERDAM, NL, PAGE(S) 323-368 , XP004170285 ISSN: 0021-9673 * page 357, right-hand column, last paragraph * * page 359, left-hand column, last paragraph - right-hand column * * page 323 - page 325 *
- DATABASE WPI Week 200033 Derwent Publications Ltd., London, GB; AN 2000-380416 XP002439001 & JP 2000 126588 A (MITSUBISHI JUKOGYO KK) 9 May 2000 (2000-05-09)
- DATABASE WPI Week 199950 Derwent Publications Ltd., London, GB; AN 1999-584200 XP002439002 & JP 11 253796 A (MITSUBISHI) 21 September 1999 (1999-09-21)
- MATSUMOTO J. ET AL.: 'Effect of laser pulse width on ionization efficiency in supersonic beam-multiphoton ionization-mass spectrometry' ANALYTICAL CHEMISTRY vol. 71, no. 17, 1999, pages 3763 - 3768, XP002956190
- ZIMMERMANN R. ET AL: 'ISOMER-SELECTIVE IONIZATION OF CHLORINATED AROMATICS WITH LASERS FOR ANALYTICAL TIME-OF-FLIGHT MASS SPECTROMETRY: FIRST RESULTS FOR POLYCHLORINATED DIBENZO-P-DIOXINS (PCDD), BIPHENYLS (PCB) AND BENZENES (PCBZ)' CHEMOSPHERE, PERGAMON PRESS, OXFORD, GB LNKD- DOI:10.1016/0045-6535(94)90353-0 vol. 29, no. 9/11, 01 January 1994, pages 1877 - 1888, XP000853798 ISSN: 0045-6535
- BOESL U. ET AL: 'A HIGH-RESOLUTION TIME-OF-FLIGHT MASS SPECTROMETER WITH LASER DESORPTION AND A LASER IONIZATION SOURCE' ANALYTICAL INSTRUMENTATION, MARCEL DEKKER INC. NEW YORK, US vol. 16, no. 1, 01 January 1987, pages 151 - 171, XP001056199

## Description

### Technical Field

The present invention relates to apparatus and method for detecting organic trace components, such as PCBs and dioxins, in treatment equipment or in the environment.

### Background Art

In recent years, bans have been imposed on production and importation of PCBs (polychlorinated biphenyls: generic term for chlorinated biphenyl isomers), because of their strong toxicity. In Japan, production of PCBs started around 1954. However, the Kanemi Yusho case revealed the adverse effects of PCBs on living organisms and the environment, and in 1972, the Japanese government issued an order that production of PCB products must be stopped and PCB products must be recovered (obligation of secure storage).

A PCB is a compound produced by substituting 1 to 10 chlorine atoms for hydrogen atoms of biphenyl. Theoretically, PCBs include 209 isomers, which differ in the number and position of substituted chlorine atoms. At present, about 100 or more PCB isomers are commercially available as PCB products. PCB isomers exhibit different physical and chemical properties, different levels of stability in living organisms, and various environmental behaviors. Therefore, chemical analysis of PCBs is difficult. Additionally, PCBs cause various types of environmental pollution. PCBs are persistent organic pollutants, and are not easily decomposed in the environment. PCBs exhibit fat solubility and high bioconcentration factor. Furthermore, PCBs can migrate through the air, because of their semi-volatility. PCBs are also known to remain in an environment; e.g., in water or in living organisms.

Since PCBs are very stable in living organisms, they accumulate therein, and the thus-accumulated PCBs cause chronic intoxication (e.g., skin diseases and liver diseases), and exhibit carcinogenicity and reproductive and developmental toxicity.

Conventionally, PCBs have been widely used as insulating oil for, for example, transformers and capacitors. In order to detoxify PCBs contained therein, the present inventors have previously proposed a hydrothermal decomposition apparatus for detoxifying PCBs (see, for example, Japanese Patent Application Laid-Open (kokai) Nos. 11-253796 and 2000-126588). FIG. 32 schematically shows the structure of such a hydrothermal decomposition apparatus.

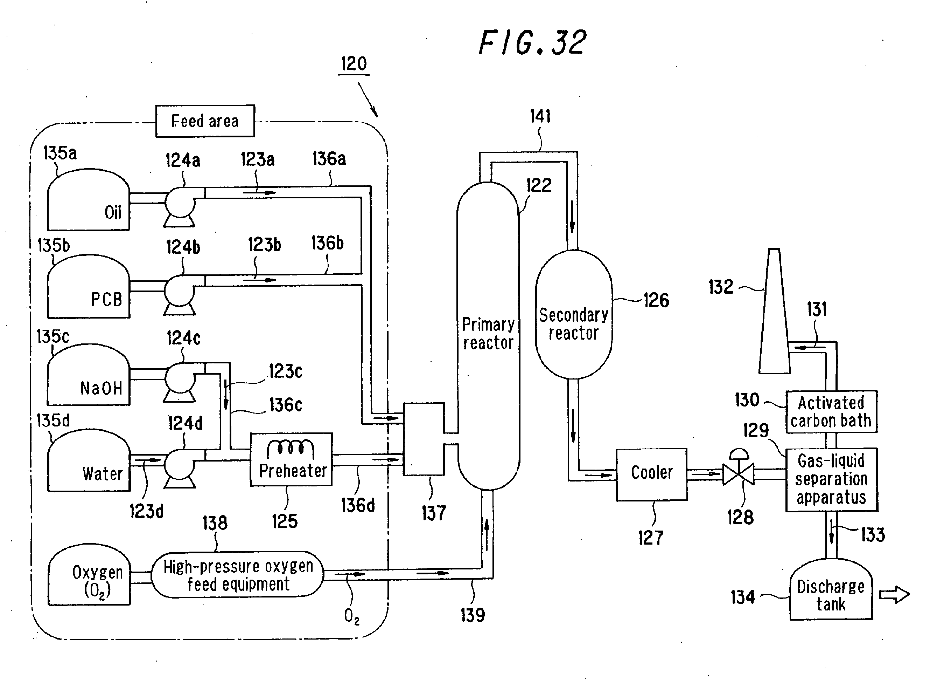

As shown in FIG. 32, a hydrothermal decomposition apparatus 120 includes a cylindrical primary reactor 122; pressurizing pumps 124a through 124d for pressurizing oil 123a, PCB 123b, NaOH 123c, and water 123d; a preheater 125 for preliminarily heating a liquid mixture of the NaOH 135c and the water 135d; a secondary reactor 126 having a spiral pipe; a cooler 127; and a pressure reduction valve 128. A gas-liquid separation apparatus 129 and an activated carbon bath 130 are provided downstream of the pressure reduction valve 128. Flue gas (CO₂) 131 is discharged from a chimney 132 to the outside, and a waste liquid (H₂O, NaCl) 133 is subjected to any treatment if desired. An oxygen feed pipe 139 is connected directly to the primary reactor 122.

In the aforementioned apparatus 120, the pressure of the interior of the primary reactor 122 is increased to 26 MPa by means of the pressurizing pumps 124. The liquid mixture 123 of PCBs, H₂O, and NaOH is preliminarily heated to about 300°C by means of the preheater 125. Oxygen is fed into the primary reactor 122, and the temperature of the interior of the primary reactor 122 is increased to 380°C to 400°C by means of heat of a reaction generated in the reactor. In the primary reactor 122, PCBs undergo dechlorination reaction and oxidative degradation reaction, and are decomposed into NaCl, CO₂, and H₂O. Subsequently, the fluid discharged from the secondary reactor 126 is cooled to about 100°C in the cooler 127, and the pressure of the fluid is reduced to atmospheric pressure by means of the pressure reduction valve 128 provided downstream of the cooler 127. Thereafter, the fluid is separated into CO₂, steam, and treated water by means of the gas-liquid separation apparatus 129. The resultant CO₂ and steam are caused to pass through the activated carbon bath 130, and are discharged to the environment.

Through treatment of PCB-containing containers (e.g., transformers and capacitors) with the aforementioned processing apparatus, complete detoxification of the containers is achieved. During this process, quick monitoring of the concentration of PCBs in the facility is critical. Conventionally, PCBs have been sampled in the form of gas and concentrated in a liquid, and the PCB-containing liquid is subjected to analysis. However, measurement of the PCB concentration by means of such a technique requires some hours to some tens of hours, and thus quick monitoring of PCB concentration is not possible.

In view of the foregoing, conventionally, there has been proposed, as an apparatus for monitoring a trace amount of PCBs contained in a gas, a mass spectrometer incorporating a multi-photon ionization detector and a time-of-flight mass spectrometer (TOFMAS).

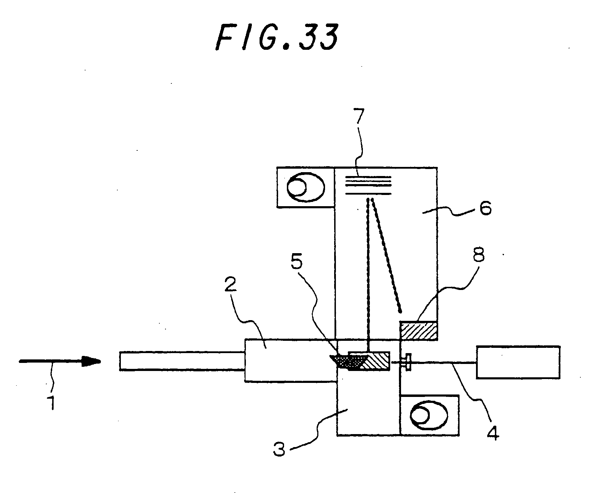

The conventional analysis apparatus will now be described with reference to FIG. 33.

As shown in FIG. 33, a sample gas 1 is fed as a supersonic free jet through a pulse nozzle 2 into a vacuum chamber 3. The free jet is cooled through adiabatic expansion. The vibrational and rotational level of the thus-cooled gas is lowered, thereby enhancing the wavelength selectivity of the gas. As a result, the gas efficiently absorbs a laser beam 4 (resonance multi-photon), and ionization efficiency of the gas is enhanced. Molecules contained in the thus-ionized gas are accelerated by means of an accelerating electrode 5, and an acceleration inversely proportional to the mass of the molecules is applied to the molecules. The thus-accelerated molecules fly through a flight tube 6. The molecules are reflected by a reflection 7, and enter a detector 8. When the flight time of the molecules in the flight tube 6 is measured, the masses of particles consisting of the molecules are calculated. The concentration of PCBs (i.e., measurement target) can be obtained through comparison of the intensities of signals output from the detector 8.

Although the aforementioned analysis apparatus can detect a trace substance, the detection sensitivity of the apparatus is low, since the apparatus employs a laser having a pulse width in the order of nanoseconds. An apparatus according to the preamble of claim 1 is described in JP 2000 321246 A. Further related art can be found in EP 1 102 004 A1.

In view of the foregoing, an object of the present invention is to provide an apparatus for detecting an organic trace component, which enable quick and highly-sensitive analysis when monitoring the concentration of PCBs contained in a gas.

In the aforementioned hydrothermal decomposition apparatus 120, various reaction parameters, including the feed amounts of various chemicals and PCBs (i.e., decomposition target), are controlled so as to decompose the PC3s completely.

Conventionally, decomposition treatment has been controlled on the basis of data obtained by measuring, for example, the amount of PCBs remaining in the treated liquid and properties of decomposition products produced during the course of decomposition treatment. However, such measurement requires some hours to one or two days.

Therefore, demand has arisen for efficient feedback control of the hydrothermal decomposition apparatus 120 during the course of decomposition treatment.

In view of the foregoing, another object of the present invention is to provide a method for controlling decomposition treatment of a toxic substance, which enables quick feedback control in an apparatus for decomposing toxic substances such as PCBs.

In use of the aforementioned apparatus for decomposing an organic halogenated substance, the concentration of PCBs in the working environment must be confirmed to be at a predetermined level or less at all times. Therefore, an apparatus for measuring a trace amount of PCBs must be calibrated at predetermined intervals so that the apparatus is operated properly.

In view of the foregoing, yet another object of the present invention is to provide the use of an organic halogenated substance concentration correction apparatus which enables quick and highly-sensitive analysis when monitoring the concentration of a trace component such as PCBs, as defined in claims 14-27.

### Disclosure of the Invention

The present invention is directed to an apparatus for detecting an organic trace component, as defined in claim 1. Further preferred embodiments are defined in the dependent claims.

### Brief Description of the Drawings

FIG. 1 is a schematic representation showing an apparatus for detecting an organic halogenated substance.
FIG. 2 shows results of measurement of low-chlorine PCBs.
FIG. 3 is a schematic representation showing a PCB concentration measuring system.
FIG. 4 shows the relation between the wavelength, pulse width, and energy density of a laser beam employed in the present invention.
FIG. 5 shows results of measurement of low-chlorine PCBs in the case where a laser beam having an energy density of 0.05 GW/cm² is employed.
FIG. 6 shows results of measurement of low-chlorine PCBs in the case where a laser beam having an energy density of 0.5 GW/cm² is employed (not an embodiment of the present invention.)
FIG. 7 is a schematic representation showing an apparatus for detecting an organic trace component according to an embodiment.
FIG. 8 shows Raman effects of a laser beam which has passed through a Raman cell.
FIG. 9 shows the relation between the wavelength and energy of a laser beam under Raman effects.
FIG. 10 shows the relation between the wavelengths and ionization efficiencies of PCBs having 1 to 6 chlorine atoms.
FIG. 11 shows results of measurement of PCBs in the case where a laser beam which has passed through a Raman cell is employed.
FIG. 12 is a schematic representation showing an apparatus used, for detecting an organic trace component according to another embodiment.
FIG. 13 is a schematic representation showing an ion trap.
FIG. 14 shows the relation between electric potential and ions approaching the center portion of the ion trap.
FIG. 15 is a schematic representation showing an apparatus for detecting an organic trace component according to a further embodiment.
FIG. 16 shows results of PCB analysis performed by use of the apparatus above in which the vacuum is regulated to 1.333 × 10⁻³ Pa (1 × 10⁻⁵ torr).
FIG. 17 shows results of comparative PCB analysis by use of the apparatus in which the vacuum is regulated to 1.333 × 10⁻² Pa (1 × 10⁻⁴ torr).
FIG. 18 is a schematic representation showing an apparatus for detecting an organic halogenated substance
FIG. 19 is a schematic representation showing multiple reflection of a laser beam.
FIG. 20 is a schematic representation showing an optical apparatus for introducing a laser beam.
FIG. 21(A) is a side view of the apparatus shown in FIG. 20; and FIG 21(B) is a plan view of the apparatus shown in FIG. 20.
FIG. 26 is a schematic representation showing an organic halogenated substance concentration correction apparatus.
FIG. 27 is a schematic representation showing an essential portion of the organic halogenated substance concentration correction apparatus.
FIG. 28 is a schematic representation showing a standard container.
FIG. 29 is a schematic representation showing another standard container.
FIG. 30 is a schematic representation showing yet another standard container.
FIG. 31 is a chart showing results of measurement of PCBs after standard calibration is performed.
FIG. 32 is a schematic representation showing a hydrothermal decomposition apparatus.
FIG. 33 is a schematic representation showing a conventional measuring apparatus employing a laser beam.

### Best Mode for Carrying Out the Invention

In order to better illustrate the present invention, the best modes thereof will next be described with reference to the appended drawings. However, the present invention is not limited only to the embodiments described below.

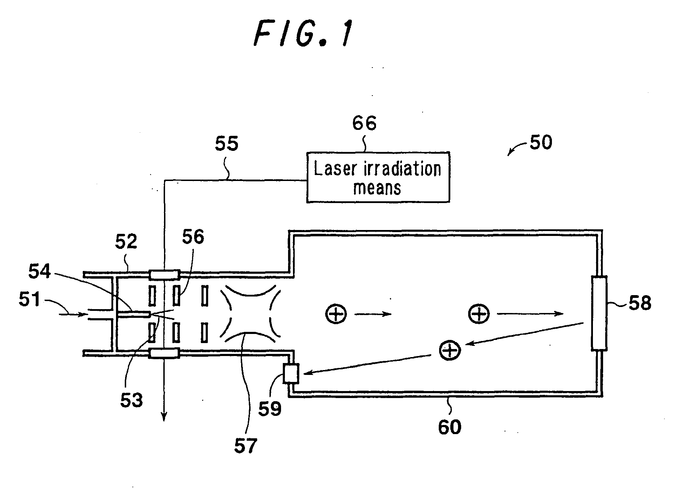

FIG. 1 is a schematic representation showing an apparatus for detecting an organic halogenated substance comprising a polychlorinated biphenyl having 1 to 3 chlorine atoms according to an embodiment of the present invention. An organic halogenated substance detection apparatus 50 of the present invention is employed for detecting an organic halogenated substance contained in a gas. As shown in FIG. 1, the detection apparatus 50 includes a capillary column 54 (sample introduction means) for continuously introducing a collected sample 51 in the form of a leakage molecular beam 53 into a vacuum chamber 52; laser irradiation means 66 for irradiating the leakage molecular beam 53 with a laser beam 55 to thereby perform ionization; a convergence section 56 for converging molecules ionized through laser irradiation, the section including a plurality of ion electrodes; an ion trap 57 for selectively trapping the thus-converged molecules; and a time-of-flight mass spectrometer 60 including an ion detector 59 for detecting ions which are emitted at predetermined intervals and reflected by a reflectron 58.

The concentration of PCBs (i.e., measurement target) can be obtained through comparison of the intensities of signals output from the detector 59.

The thus-obtained PCB concentration data may be sent to, for example, a monitor-control chamber, and to the outside by means of, for example, a monitoring apparatus (not illustrated) provided outside the detection apparatus 50.

Preferably, the capillary column 54 is provided such that the tip of the column projects into the ion convergence section 56. Specifically, the tip of the capillary column is made to be flush with an electrode constituting the ion convergence section 56 that is adjacent to the capillary column; or the capillary column is provided such that the tip thereof projects into the convergence section toward the ion trap by a certain length as measured from the electrode.

Preferably, the capillary column is formed of quartz or stainless steel. When the capillary column is formed of stainless steel, the leakage molecular beam can be regulated under application of voltage to the ion convergence section 56.

The diameter of the capillary column is preferably 1 mm or less. Preferably, the capillary column is provided such that the tip thereof is located about 3 mm away from the laser beam. Preferably, the outlet of the capillary is located a short distance away from a position at which the molecular beam is irradiated with the laser beam. However, when the distance between the outlet and the irradiation position is very small, the tip of the capillary column is broken by the laser beam. Therefore, preferably, the distance is decreased such that breakage of the tip does not occur; for example, the distance is decreased to about 1 to 2 mm, to thereby enhance ionization efficiency.

The pulse wavelength of the laser beam 55 radiated from the laser irradiation means 66 is 250 - 280 nm, preferably 266 ± 10 nm.

The pulse width of the laser beam 56 radiated from the laser irradiation means 66 is 100 to 500 picoseconds. When a laser beam having a pulse width in the order of nano (10⁻⁹)-seconds is employed, detection sensitivity is lowered.

Thus, as a laser beam having a pulse width of 100-500 pico (10⁻¹² )-seconds is employed, decomposition of PCB by the laser beam can be suppressed, and detection sensitivity can be enhanced.

Table 1 shows results of measurement of PCB detection sensitivities when laser beams having different pulse widths are employed.

This measurement employed a PCB sample containing PCBs having 1 to 4* chlorine atoms (hereinafter PCB having n chlorine atoms may be simply referred to as "n-Cl PCB") and predominantly containing 2-Cl PCB and 3-Cl PCB.

Signal intensities were measured by use of a laser beam having a pulse width of 100 picoseconds and a laser beam having a pulse width of 50 nanoseconds.

In the present invention, a pico-second laser (fixed wavelength: 266 nm) was employed for measurement.

**[Table 1] PCB detection sensitivities at different laser pulse widths**

| Measurement, molecule | Signal intensity (mV) when a laser beam having a pulse width of 100 picoseconds is employed | Signal intensity (mV) when a laser beam having a pulse width of 50 nanoseconds is employed |
|---|---|---|
| 1-Cl PCB | 51.5 | 6.8 |
| 2-Cl PCB | 87.5 | 12.8 |
| 3-Cl PCB | 60.8 | 6.4 |
| 4-Cl PCB* | 1.7 | 0.4 |
| PCB decomposition products | 50.4 | 123.4 |

| | | |
|---|---|---|
| *Reference-not within the scope of the invention | | |

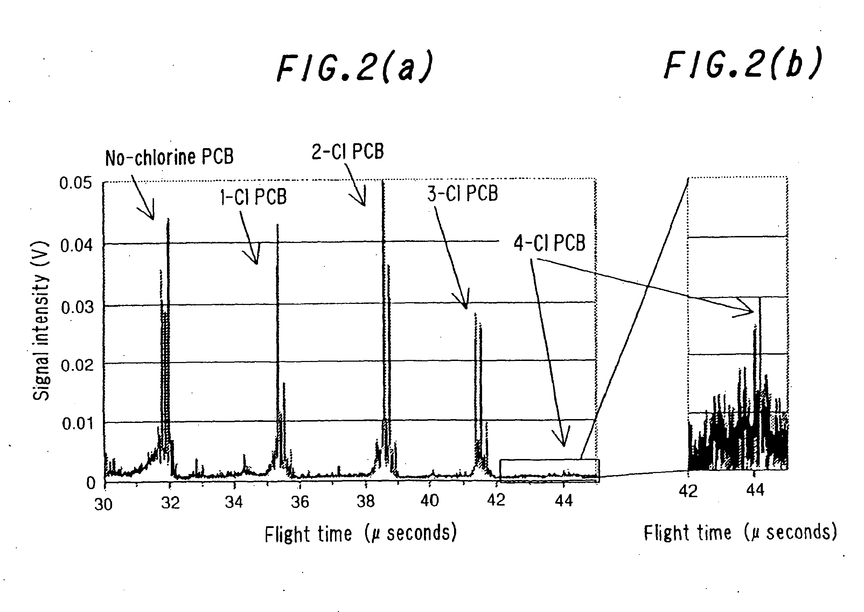

FIGs. 2 (a) and 2 (b) are charts showing signals output from the ion detector which detected PCBs. In each of the charts, the horizontal axis represents flight time (seconds) and the vertical axis represents ion signal intensity (V). The signal corresponding to 4-Cl PCB is also shown in FIG. 2(b), which is an enlarged view of FIG. 2(a).

Through use of the aforementioned measuring apparatus, the concentration of PCBs remaining in, for example, a gas in PCB decomposition treatment equipment or waste liquid discharged from the equipment can be measured quickly and accurately. Monitoring of, for example, a treatment process can be performed on the basis of the measurement results.

When the concentration of PCBs remaining in waste liquid is measured, the waste liquid is introduced into the measuring apparatus, or the waste liquid is vaporized and the resultant vapor is introduced into the apparatus.

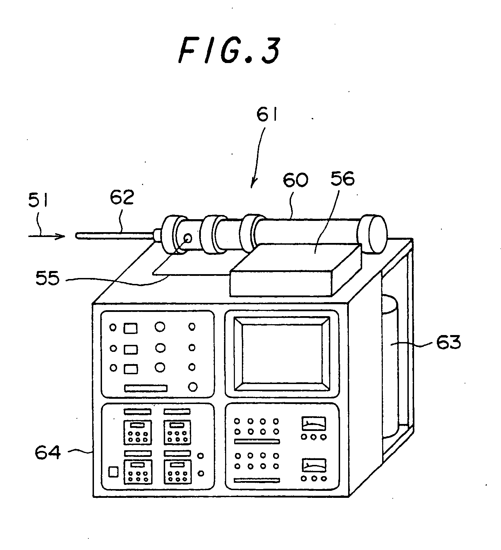

FIG. 3 is a schematic representation showing an apparatus for measuring the concentration of PCB contained in a gas.

As shown in FIG. 3, a PCB concentration measuring system 61 includes a gas sampling line 62 which is connected to the vacuum chamber 52 of the detection apparatus 50. As shown in FIG. 1, a sample is introduced as a leakage molecular beam into the vacuum chamber 52 through the line 62 and ionized by the laser beam 55 radiated from the laser irradiation means 66, and the resultant ions are detected by the time-of-flight mass spectrometer 60. In FIG. 3, reference numeral 63 denotes an evacuation apparatus for evacuating the vacuum chamber 52, and reference numeral 64 denotes a controller for controlling the aforementioned apparatuses.

Through use of the measuring system 61, quick and highly-sensitive PCB analysis can be performed; specifically, PCB can be detected at a sensitivity of 0.01 mg/Nm³ within one minute.

The wavelength of the laser beam 55 radiated from the laser irradiation means 66 is 250-280 nm, preferably 266 ± 10 nm.

In the present embodiment, the pulse width (laser pulse width) of the laser beam 55 radiated from the laser irradiation means 66 is 100 to 500 pico (10⁻¹²)-seconds (ps). When a laser beam having a pulse width in the order of nano (10⁻⁹)-seconds is employed, detection sensitivity is lowered.

The energy density (GW/cm²) of the laser beam 55 radiated from the laser irradiation means 66 is preferably 0.1 to 0.01 GW/cm², more preferably 0.05 to 0.01 GW/cm². When the laser energy density (GW/cm²) exceeds 1 GW/cm², the amount of PCB decomposition products increases.

As described above, in the present invention, the wavelength of the laser beam is regulated to 250-280 nm the pulse width of the laser beam is regulated to 100 to 500 picoseconds, and the laser energy density is regulated to 0.1 to 0.01 GW/cm². Thus, decomposition of PCB by the laser beam can be suppressed, and detection sensitivity can be greatly enhanced.

Particularly preferably, the energy density is regulated to 0.1 GW/cm² or thereabouts.

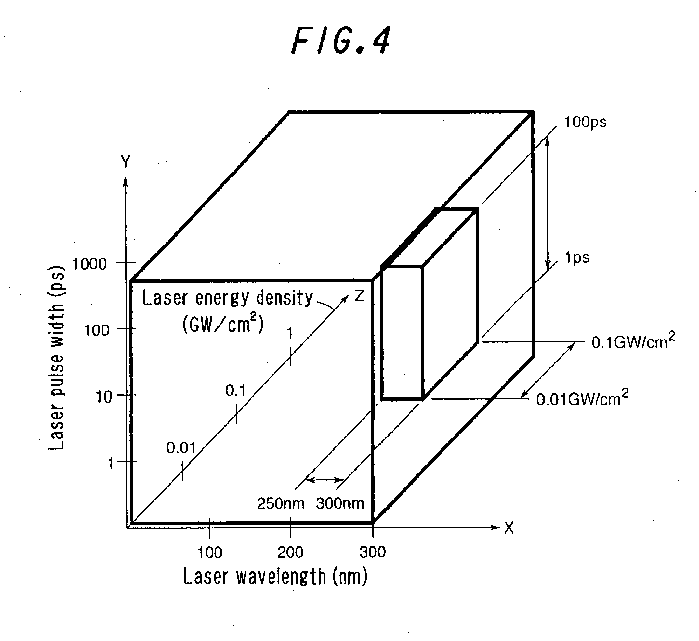

FIG. 4 shows the relation between the aforementioned wavelength, pulse width, and energy density.

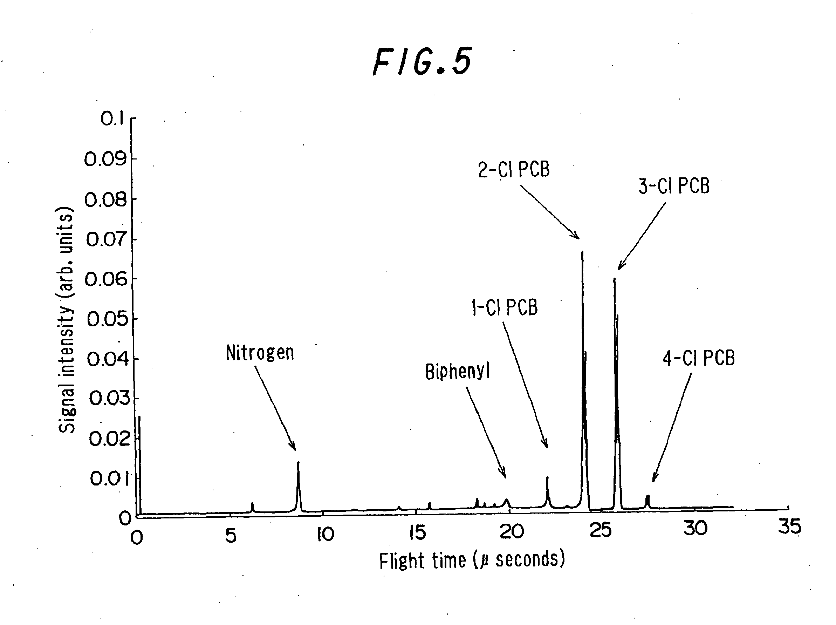

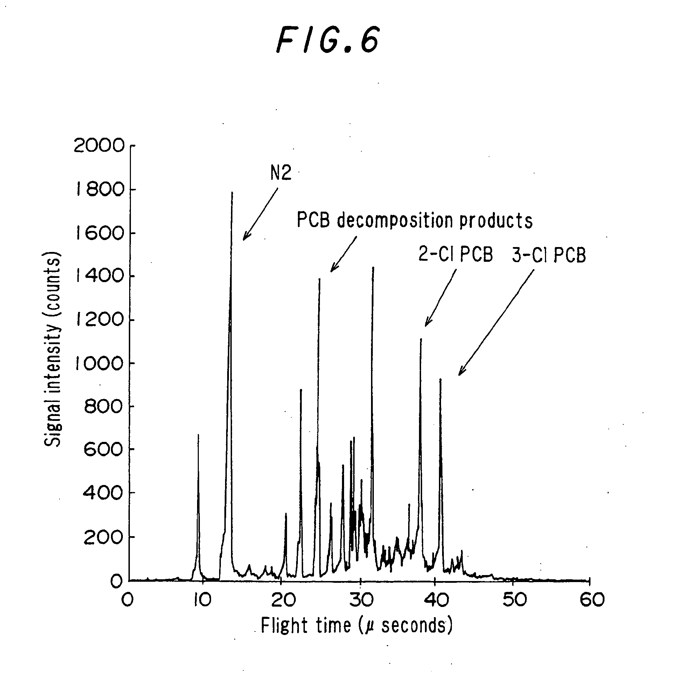

Mass spectra of a PCB standard sample and an N₂ gas sample were measured by use of the time-of-flight mass spectrometer. The results are shown in FIGs. 5 and 6. In this measurement, a laser beam having a pulse width of 100 picoseconds was employed. FIG. 5 shows measurement results for the case where the energy density of the laser beam is 0.05 GW/cm², and FIG. 6 shows measurement results for the case where the energy density of the laser beam is 0.5 GW/cm². (not in accordance with the invention).

In this measurement, a PCB sample containing 1- to 4-Cl PCBs and predominantly containing 2-Cl PCB and 3-Cl PCB was employed.

As shown in FIG. 5, in the case where the energy density of the laser beam is 0.05 GW/cm², clear peaks corresponding to PCBs are obtained. In contrast, as shown in FIG. 6, in the case where the energy density of the laser beam is 0.5 GW/cm², large amounts of PCB decomposition products are generated, and clear peaks corresponding to PCBs are not obtained.

Through use of the aforementioned measuring apparatus, the concentration of PCB remaining in, for example, a gas in PCB decomposition treatment equipment can be measured quickly and accurately at high sensitivity. Monitoring of a treatment process can be performed on the basis of the measurement results.

In another embodiment, the laser irradiation conditions are further specified; specifically, a laser beam which has passed through a Raman cell is employed.

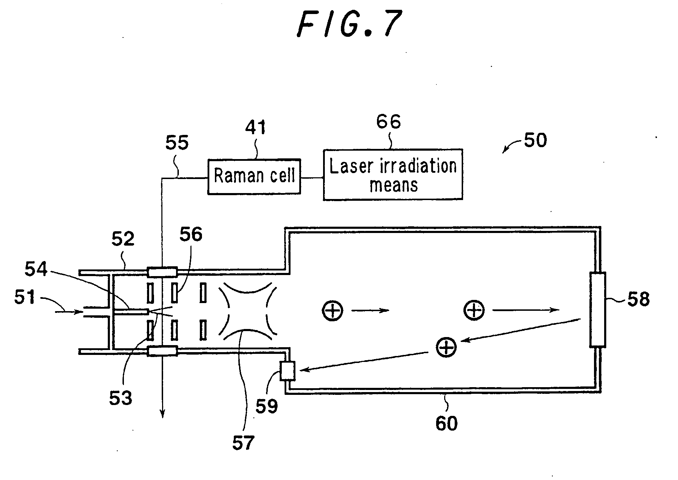

FIG. 7 is a schematic representation showing an organic trace component detection apparatus according to the present embodiment. An organic trace component detection apparatus 50 of the present embodiment is employed for detecting an organic trace component contained in waste liquid or flue gas. As shown in FIG. 7, the detection apparatus 50 includes a capillary column 54 (sample introduction means) for continuously introducing a collected sample 51 in the form of a leakage molecular beam 53 into a vacuum chamber 52; laser irradiation means 66 for irradiating the leakage molecular beam 53 with a laser beam 55 which has passed through a Raman cell 41 to thereby perform ionization; a convergence section 56 for converging molecules ionized through laser irradiation, the section including a plurality of ion electrodes; an ion trap 57 for selectively trapping the thus-converged molecules; and a time-of-flight mass spectrometer 60 including an ion detector 59 for detecting ions which are emitted at predetermined intervals and reflected by a reflectron 58.

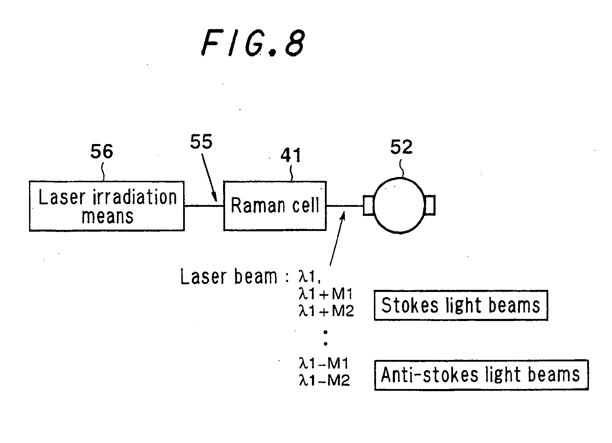

When the Raman cell 41 is provided, as shown in FIG. 8, the laser beam 55 (wavelength: λ₁) radiated from the laser irradiation means 66 is separated into Stokes light beams (wavelength: λ₁ + M₁, λ₁ + M₂ · · ·) and anti-Stokes light beams (wavelength: λ₁ - M₁, λ₁ - M₂ · · · ). Thus, a plurality of light beams having different wavelengths are obtained from the laser beam 55 having a single wavelength. Therefore, PCBs having different numbers of substituted chlorine atoms can be simultaneously excited by the thus-obtained light beams of different wavelengths.

Examples of the aforementioned Raman cell include a Raman cell containing a gas such as N₂, H₂, or CH₄ at high pressure (e.g., about 50 atm). When a laser beam of 266 nm is introduced into such a Raman cell, through interaction between molecules of a gas contained in the cell, a light beam having a specific wavelength is emitted from the cell; for example, a light beam of 283 nm is emitted in the case where the cell contains N₂, a light beam of 301 nm is emitted in the case where the cell contains H₂, or a light beam of 288 nm is emitted in the case where the cell contains CH₄.

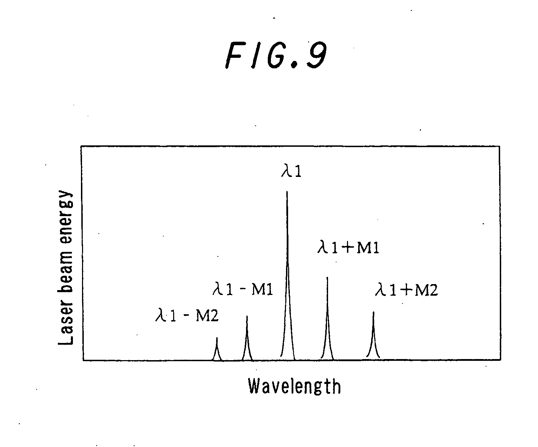

FIG. 9 shows the relation between the wavelengths and energies of light beams obtained when the laser beam 55 passes through the Raman cell 41.

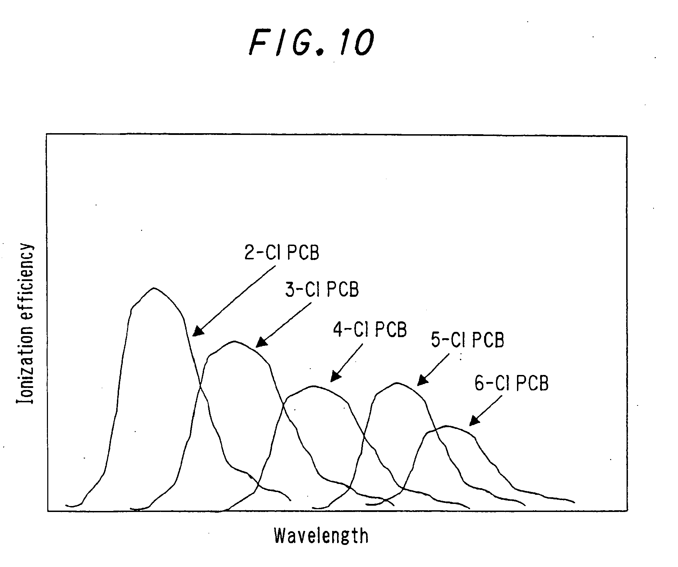

As shown in FIG. 10, when the number of substituted chlorine atoms of PCB increases, the absorption wavelength of the PCB shifts from a low level to a high level. Therefore, the concentrations of PCBs can be efficiently measured by use of a plurality of light beams having different wavelengths obtained from a laser beam which has passed through the Raman cell 41.

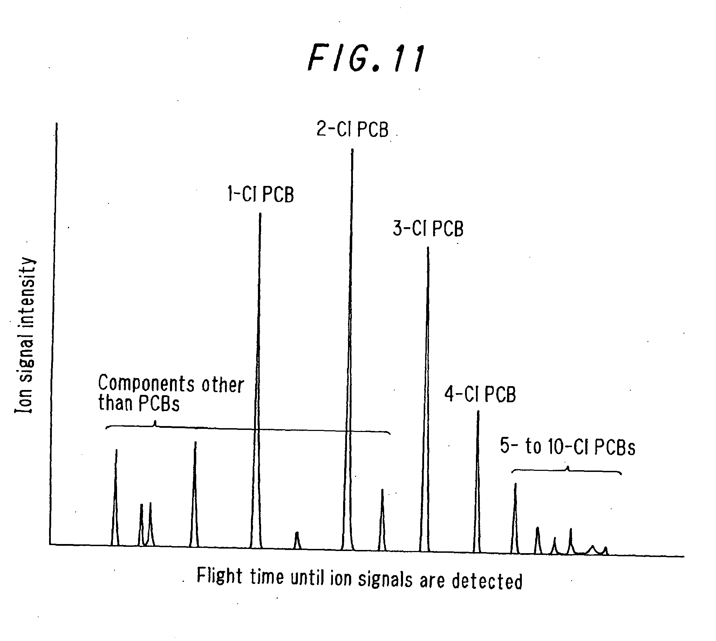

FIG. 11 shows results of measurement of PCBs by use of the apparatus shown in FIG. 7, in which a laser beam is separated into light beams of different wavelengths by means of the Raman cell. In the chart of FIG. 11, the horizontal axis represents flight time (seconds) and the vertical axis represents ion signal intensity (V).

The results show that efficient measurement can be performed by use of the apparatus shown in FIG. 7.

Through use of the aforementioned measuring apparatus, the concentration of PCB remaining in, for example, waste liquid discharged from PCB decomposition treatment equipment can be measured quickly and accurately. Monitoring of a treatment process can be performed on the basis of the measurement results.

In get another embodiment, the trapping conditions for molecules ionized through laser irradiation in the first embodiment are further specified.

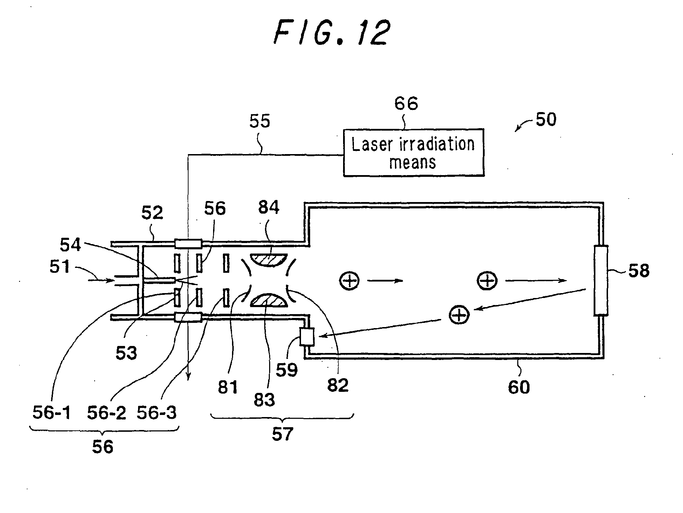

FIG. 12 is a schematic representation showing an organic trace component detection apparatus according to the present embodiment. An organic trace component detection apparatus 50 of the present embodiment is employed for detecting an organic trace component contained in waste liquid or flue gas. As shown in FIG. 12, the detection apparatus 50 includes a capillary column 54 (sample introduction means) for continuously introducing a collected sample 51 in the form of a leakage molecular beam 53 into a vacuum chamber 52; laser irradiation means 66 for irradiating the leakage molecular beam 53 with a laser beam 55 to thereby perform ionization; a convergence section 56 for converging molecules ionized through laser irradiation, the section including a plurality of ion electrodes 56-1 to 56-3; an ion trap 57 for selectively trapping the thus-converged .molecules; and a time-of-flight mass spectrometer 60 including an ion detector 59 for detecting ions which are emitted at predetermined intervals and reflected by a reflectron 58.

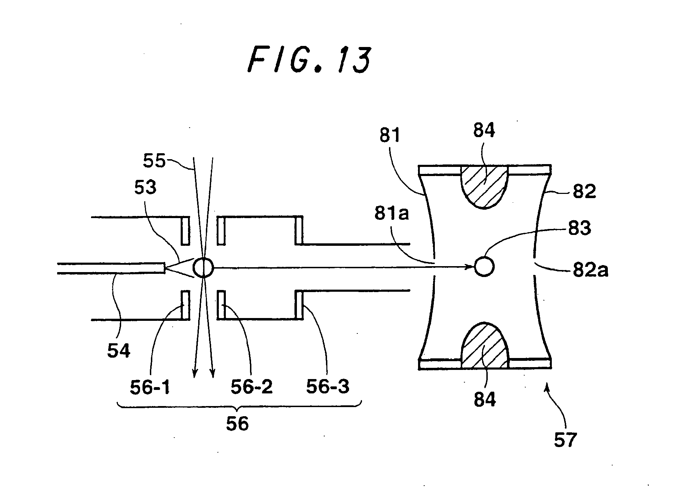

FIG. 13 is a schematic representation showing the ionization zone and the ion trap.

As shown in FIG. 13, the ion trap 57 includes a first end cap electrode 81 having a small hole 81a through which the ionized molecules enter; a second end cap electrode 82 having a small hole 82a from which the trapped molecules are emitted, the first and second end cap electrodes facing each other; and a high-frequency electrode 84 for applying high-frequency voltage to an ion trap zone 83.

The voltage of the first end cap electrode 81 is regulated to be lower than the voltage (e.g., 6 V) of the ion convergence section 56 for converging the ionized molecules; for example, the voltage of the electrode 81 is regulated to 0 V. The voltage of the second end cap electrode 82 is regulated to be higher than the voltage (e.g., 0 V) of the first end cap electrode 81; for example, the voltage of the electrode 82 is regulated to 12 V.

Since the voltage of the first end cap electrode 81 is regulated to be lower than the voltage (e.g., 6 V) of the ion convergence section 56 for converging the ionized molecules; for example, the voltage of the electrode 81 is regulated to 0 V, the ionized molecules are accelerated toward the first end cap electrode 81, and the molecules pass through the small hole 81a of the first end cap electrode 81 and efficiently enter the ion trap 57. The molecules are rapidly decelerated in the ion trap 57, since the voltage of the second end cap electrode 82 is regulated to be higher than the voltage (e.g., 0 V) of the first end cap electrode 81; for example, the voltage of the electrode 82 is regulated to 12 V. When high-frequency voltage is applied to the ion trap 57 by means of the high-frequency electrode 84, the molecules are rotated and trapped in the vicinity of the center of the ion trap 57.

After application of the high-frequency voltage by means of the high-frequency electrode 84 is stopped, when a high voltage (e.g., 400 V) is applied to the first end cap electrode 81 and a low voltage (e.g., -400 V) is applied to the second end cap electrode 82, the above trapped ions are emitted from the small hole 82a and are detected by the ion detector 59 provided in the time-of-flight mass spectrometer 60.

The concentration of a measurement target (e.g., PCBs) can be obtained through comparison of the intensities of signals output from the detector 59.

In the present invention, preferably, the voltage and frequency of the high-frequency electrode are regulated to 1,000 to 1,500 V and at least 1 MHz, respectively. In the case where PCBs are the targets of measurement, when the voltage and frequency are regulated to the above values, efficient trapping of ions is achieved in the ion trap zone.

No particular limitations are imposed on the voltage and frequency of the high-frequency electrode, since the voltage and frequency can be appropriately varied in accordance with the shape of the ion trap and the type of the measurement target, thereby optimizing conditions for ion trapping.

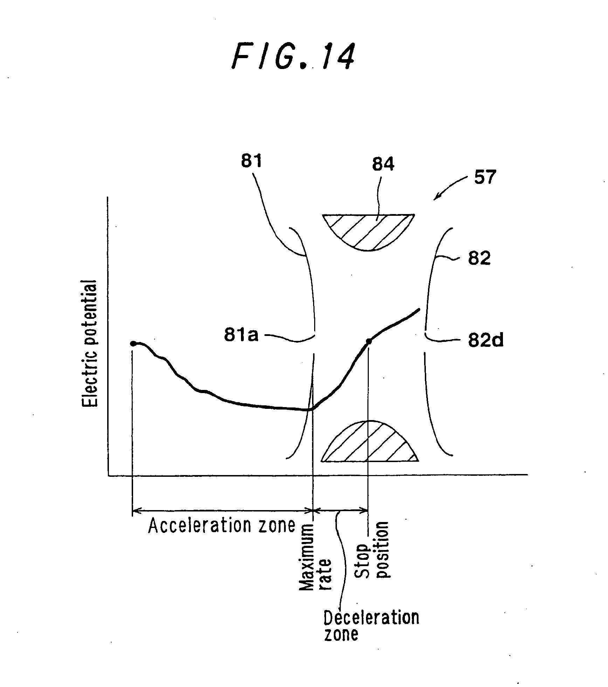

FIG. 14 shows the relation between electric potential and ions approaching the center portion of the ion trap in the case where the voltages of the first electrode 56-1, the second electrode 56-2, and the third electrode 56-3 are 6V, 6V, and 5 V, respectively; the voltage of the first end cap electrode 81 is 0 V; and the voltage of the second end cap electrode 82 is 12 V.

As shown in FIG. 14, ionized molecules are accelerated by means of the lensing effect of the convergence section 56, the velocity of the molecules becomes maximum at the first end cap electrode 81, and the thus-accelerated molecules pass through the small hole 81a of the first end cap electrode 81. The molecules are rapidly decelerated in the ion trap, since the voltage of the second end cap electrode 82 is 12V; i.e., the voltage of the electrode 82 is higher than that of the electrode 81. As a result, motion of the molecules stops in the vicinity of the center of the ion trap 57.

Motion of the trapped molecules stops in the vicinity of a position at which an electric potential is nearly equal to that of a position at which ionization of the molecules has been initiated. Therefore, the voltage of the second end cap electrode 82 may be determined so as to regulate the stop position of the trapped molecules.

Preferably, the voltage of the second end cap electrode 82 is regulated to become about twice that of the first electrode 56-1. In the present embodiment, the voltage of the first electrode 56-1 is regulated to 6V, and the voltage of the second end cap electrode 82 is regulated to 12 V.

The voltage of the second end cap electrode is not necessarily regulated to become twice that of the first electrode 56-1, and the voltage of the second end cap electrode may be optionally determined in accordance with the shape of the ion trap and the mass of molecules to be trapped.

As described above, in order to stop motion of the ionized sample in the ion trap, the voltage of the second end cap electrode 82 must be regulated to become higher than that of the first end cap electrode 81.

No particular limitations are imposed on the means for ionizing a sample. For example, the ionization means may be laser irradiation means for irradiating a sample with a laser beam to thereby ionize the sample. Alternatively, a sample may be ionized by use of, for example, an electron gun or plasma.

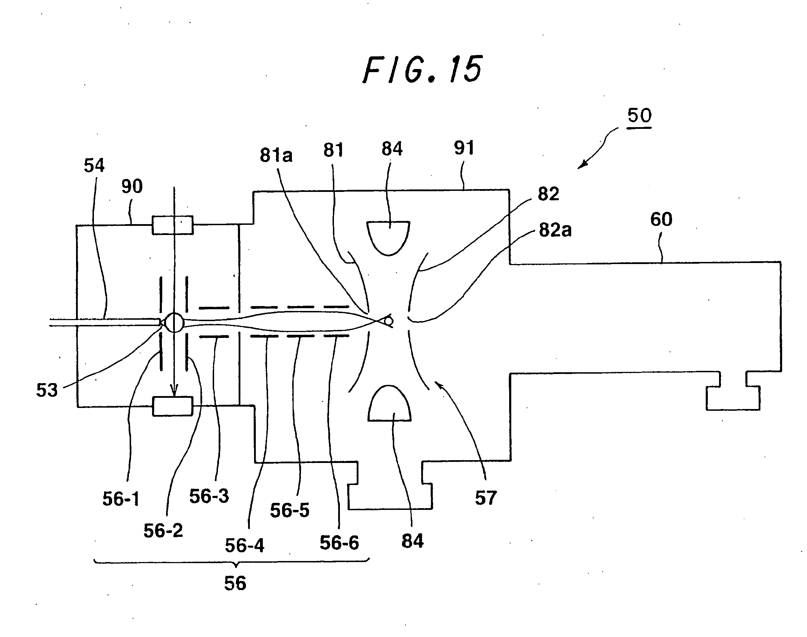

FIG. 15 is a schematic representation showing an organic trace component detection apparatus according to a further embodiment. An organic trace component detection apparatus 50 of the present embodiment is employed for detecting an organic trace component contained in waste liquid or flue gas. As shown in FIG. 15, the detection apparatus 50 includes an ionization zone 90 for ionizing a sample; a zone 91 including an ion convergence section 56 for converging ionized molecules and accelerating the molecules toward an ion trap 57, and the ion trap 57 for trapping ions; and a time-of-flight mass spectrometer 60. The ionization zone 90, the zone 91, and the spectrometer 60 are separated from one another by means of partition walls. The vacuum of the ionization zone 90 is regulated to 1 × 10⁻³ torr, the vacuum of the zone 91 including the ion convergence section and the ion trap is regulated to 1 × 10⁻⁵ torr, and the vacuum of the time-of-flight mass spectrometer 60 is regulated to 1 × 10⁻⁷ torr.

Components of the above apparatus that are similar to those employed in the apparatus shown in FIGs. 13 and 14 are denoted by common reference numerals, and repeated descriptions thereof are omitted.

In the present embodiment, the ionization zone 90 and the zone 91 including the ion convergence section and the ion trap are separated from each other. Therefore, unwanted gas is not introduced into the zone 91 including the ion convergence section and the ion trap, and thus efficient analysis is performed.

Since the vacuum of the zone 91 including the ion convergence section and the ion trap is regulated to as low as 1 × 10⁻⁵ torr, the amount of an inert gas can be reduced, and decomposition of a target substance which is readily decomposed can be prevented.

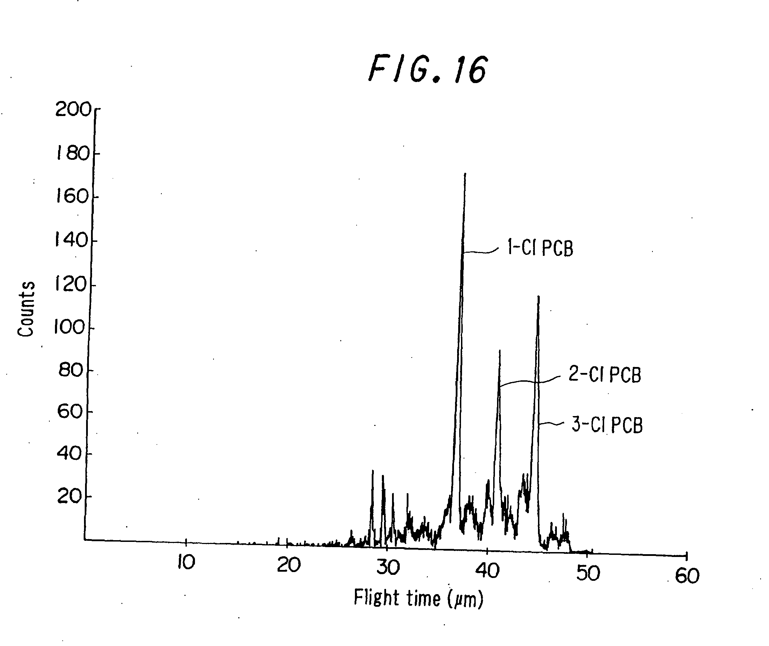

FIG. 16 shows results of measurement of PCBs in the case where the vacuum of the zone 91 including the ion convergence section and the ion trap was regulated to 1 × 10⁻⁵ torr. As shown in FIG. 16, clear peaks corresponding to 1-Cl PCB, 2-Cl PCB, and 3-Cl PCB were obtained.

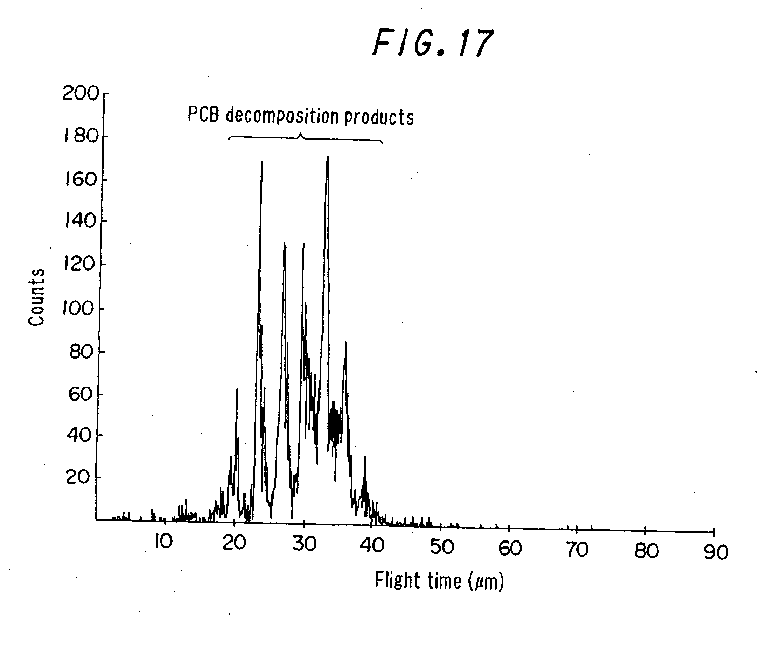

FIG. 17 shows results of measurement of PCBs in the case where the vacuum of the zone 91 including the ion convergence section and the ion trap was regulated to 1 × 10⁻⁴ torr.

As shown in FIG. 17, clear peaks corresponding to PCBs were not obtained; the peaks correspond to PCB decomposition products.

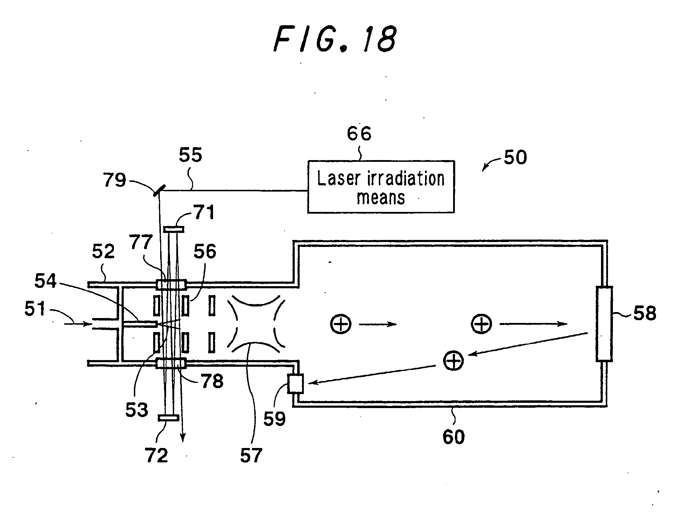

FIG. 18 is a schematic representation showing a laser-type measuring apparatus according to get another embodiment. As shown in FIG. 18, a laser-type measuring apparatus 50 of the present embodiment includes a capillary column 54 (sample introduction means) for continuously introducing a collected sample 51 in the form of a leakage molecular beam 53 into a vacuum chamber 52; laser irradiation means 66 for irradiating the leakage molecular beam 53 with a laser beam 55 to thereby perform ionization; a convergence section 56 for converging molecules ionized through laser irradiation, the section including a plurality of ion electrodes; an ion trap 57 for selectively trapping the thus-converged molecules; and a time-of-flight mass spectrometer 60 including an ion detector 59 for detecting ions which are emitted at predetermined intervals and reflected by a reflectron 58. The concentration of a measurement target can be obtained through comparison of the intensities of signals output from the detector 59.

In FIG. 18, reference numerals 77 and 78 denote lens windows, and reference numeral 79 denotes a reflection mirror.

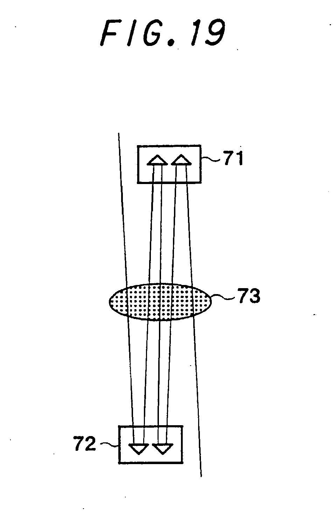

As shown in FIG. 19, the laser beam 55 radiated from the laser irradiation means 66 is repeatedly reflected by means of facing prism means 71 and 72 such that the thus-reflected laser beams do not overlap one another within an ionization zone 73, and a sample introduced into the zone 73 is irradiated with the laser beams. The prism means 71 incorporates a plurality of prisms, but no particular limitations are imposed on the prism means.

Since a plurality of pulse laser beams do not simultaneously impinge on the same portion of the introduced sample, decomposition of the sample is prevented. In addition, efficiency in ionization of the sample through laser irradiation is enhanced.

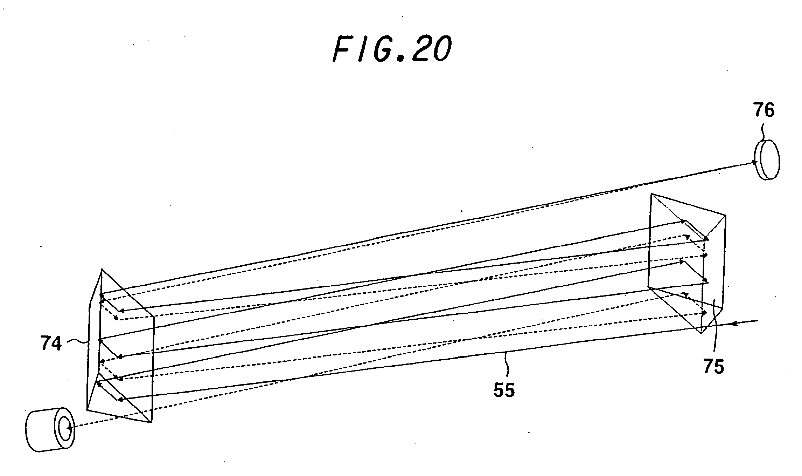

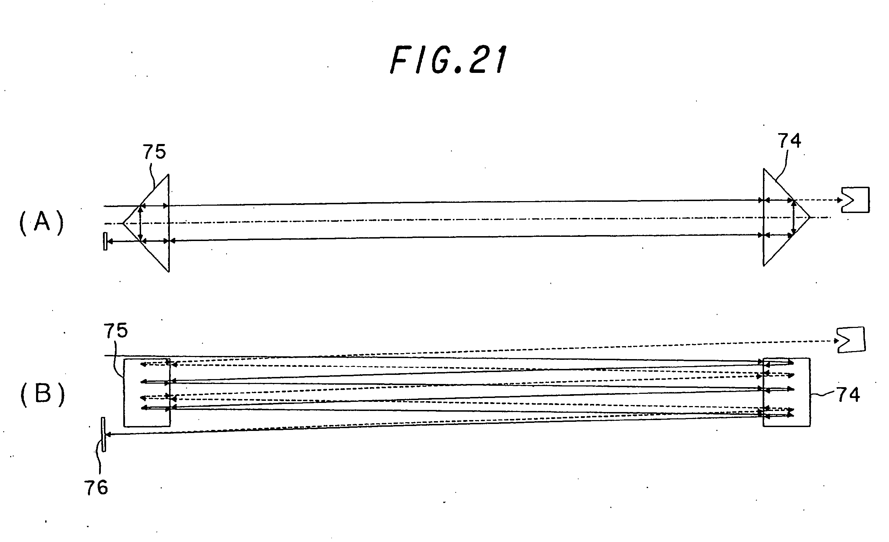

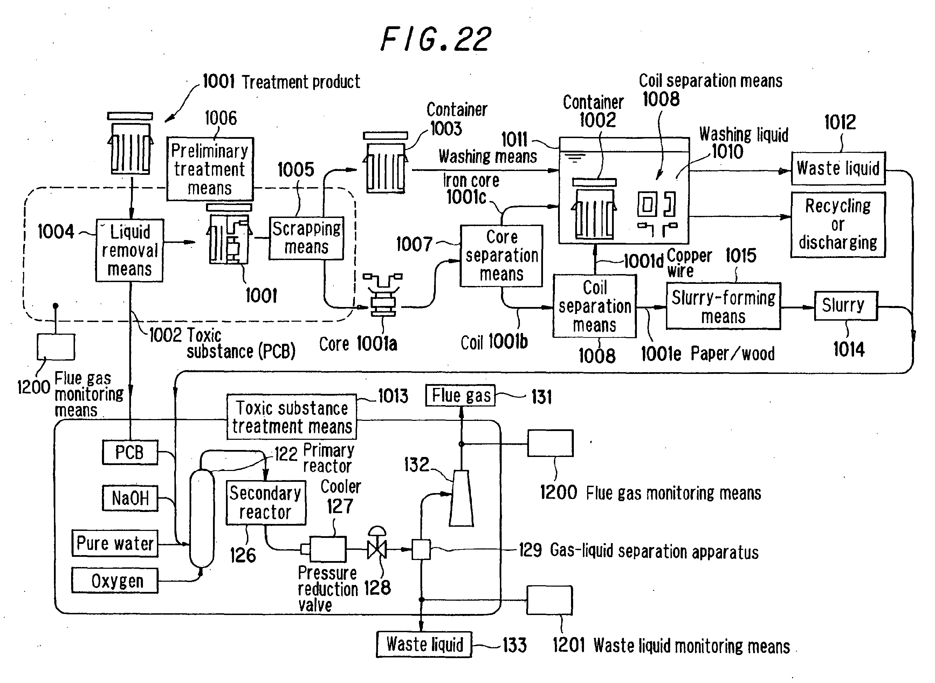

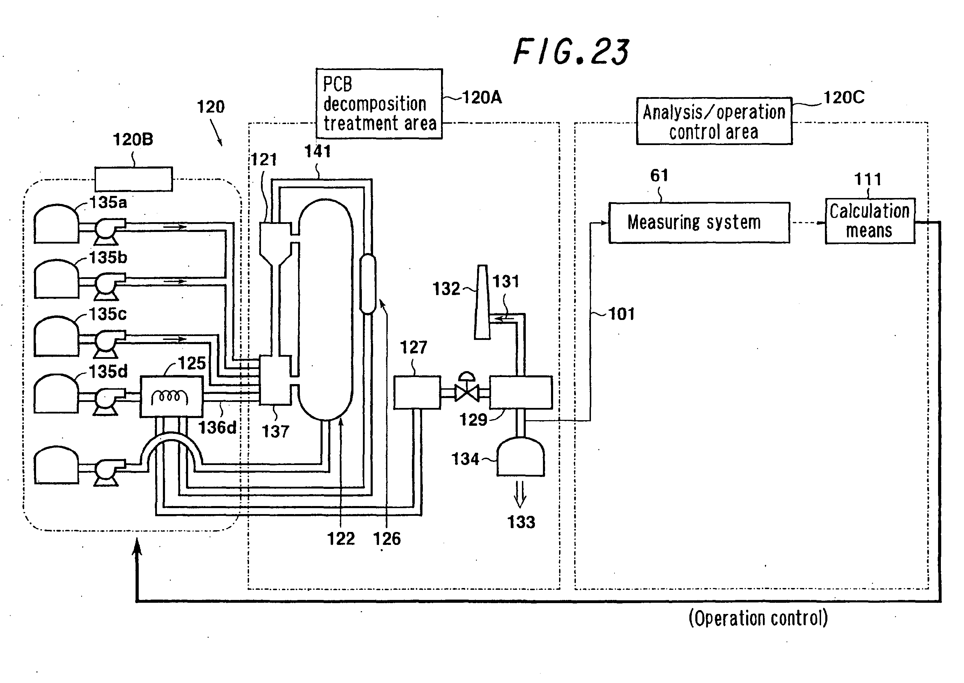

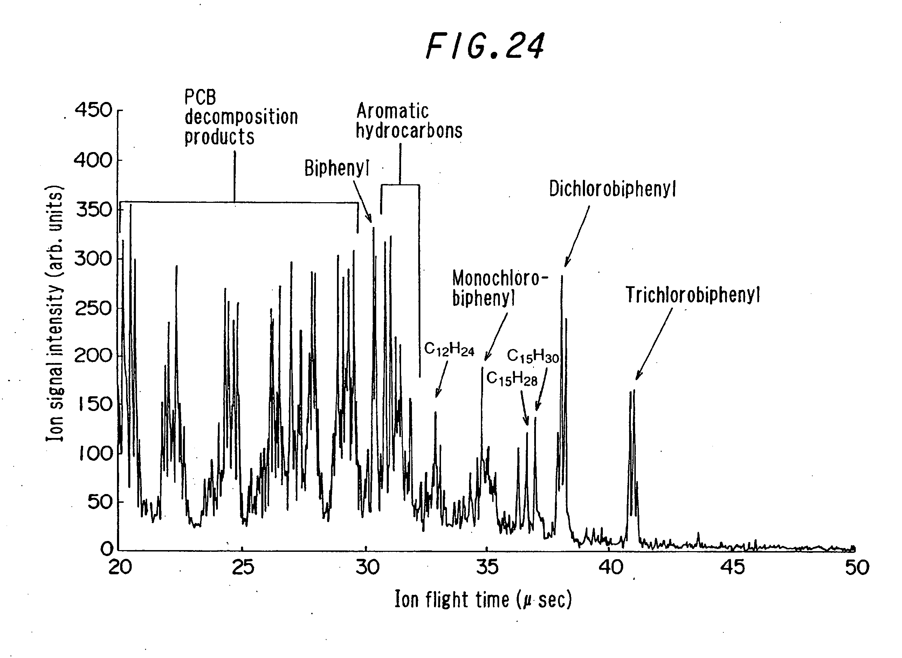

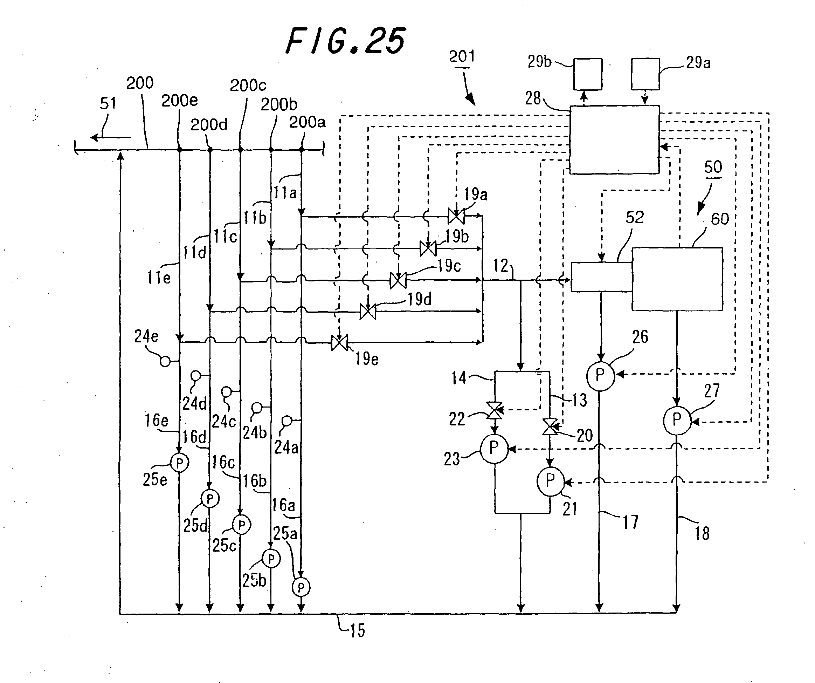

FIGs. 20 and 21 schematically show an optical apparatus for introducing laser beams so as to prevent overlapping of the laser beams. FIG. 20 is a perspective view of the optical apparatus; FIG. 21(A) is a side view of the apparatus; and FIG. 21(B) is a plan view of the apparatus.

As shown in FIGs. 20 and 21, the optical apparatus includes prisms 74 and 75 which face each other. When the incident angle of the laser beam 55 is regulated, the reflected laser beams do not overlap one another. In the present embodiment, since a reflection mirror 76 is provided, a sample is repeatedly excited by the reflected laser beams.

In the case where laser irradiation is performed within an ionization zone of 6 mm, when a laser beam of 1 mJ is reflected 10 times by means of the aforementioned prisms, the same effect is obtained as in the case where a laser beam of 10 mJ is employed. Therefore, costs required for the measuring apparatus can be reduced.

In the case where a laser beam is repeatedly reflected, when the thus-reflected laser beams overlap one another, molecules of a measurement target that have been ionized by a laser beam are irradiated again with another laser beam, thereby promoting decomposition of the molecules.

In the present invention, a laser beam is repeatedly reflected such that the thus-reflected laser beams do not overlap one another.

In order to repeatedly reflect a laser beam such that the thus-reflected laser beams do not overlap one another, for example, a technique as shown in FIG. 19 is employed; i.e., a technique employing a plurality of prisms and a refection mirror. Alternatively, there may be employed a technique in which an incident laser beam is regulated by use of a pair of facing prisms. Any technique may be employed in the present invention, so long as a laser beam can be repeatedly reflected such that the thus-reflected laser beams do not overlap one another.

When trace components are continuously measured over a long period of time by means of the aforementioned monitoring system, a correction apparatus must be employed. An example of a correction apparatus will now be described in connection with the present embodiment.

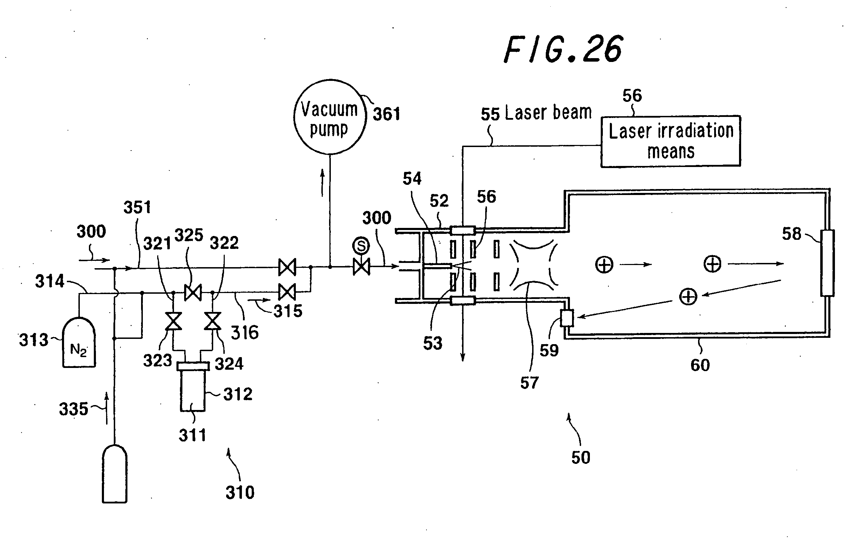

FIG. 26 is a schematic representation showing an organic halogenated substance concentration correction apparatus used according to an embodiment. As shown in FIG. 26, an organic halogenated substance concentration correction apparatus 310 used according to the present embodiment includes a standard container 312 containing an organic halogenated substance 311 of predetermined concentration; a purge gas feed tube 314 for feeding a purge gas 313 for purging the organic halogenated substance 311 contained in the standard container 312; and a standard gas introduction tube 316 for introducing into a mass spectrometer 50 a standard gas 315 containing the organic halogenated substance 311 of predetermined concentration which is accompanied by the purge gas 313.

The purge gas feed tube 314 has a branched feed line 321 for feeding the purge gas into the standard container. Valves 323 and 324 are provided on the feed line 321 and a gas discharge line 322, respectively. A valve 325 is provided on a path between the purge gas feed tube 314 and the standard gas introduction tube 316 so as to effect opening and closure of the path.

The temperature of the interior of the standard container 312 is maintained at a temperature 5 to 30 degrees higher than the temperature of an atmosphere surrounding the container, by means of temperature-maintaining means (not illustrated), to thereby maintain the saturation concentration of the organic halogenated substance 311 contained in the container.

Table 2 shows the relation between saturation vapor pressure and PCB concentration in the case where the aforementioned organic halogenated substance 311 is PCBs.

When the aforementioned halogenated substance is 2- to 4-Cl PCBs, preferably, the temperature of the standard container is maintained at 35°C (i.e., room temperature (25°C) + 10 degrees) by means of a thermostatic bath.

When the aforementioned halogenated substance is 5- to 7-Cl PCBs, preferably, the temperature of the standard container is maintained at 50°C (i.e., room temperature (25°C) + 25 degrees) by means of a thermostatic bath.

**[Table 2]**

| Saturation vapor pressure | PCB concentration |
|---|---|
| 10 mg/Nm³ | 2-Cl PCB · · · 0.2 mg/Nm³ |
| 3 mg/Nm³ | 3-Cl PCB · · · 0.5 mg/Nm³ |
| 0.5 g/Nm³ | 4-Cl PCB · · · 0.05 mg/Nm³ |

Kanechlor KC300 (product of Kanegafuchi Chemical Industry Co., Ltd.), a commercially available PCB product, contains 2-Cl PCB, 3-Cl PCB, and 4-Cl PCB. Kanechlor KC400 (product of Kanegafuchi Chemical Industry Co., Ltd.) contains 3-Cl PCB, 4-Cl PCB, and 5-Cl PCB. Kanechlor K60 (product of Kanegafuchi Chemical Industry Co., Ltd.) contains 5-Cl PCB, 6-Cl PCB, and 7-Cl PCB.

In order to maintain the saturation concentration of PCBs at a predetermined level, a PCB solution prepared by dissolving PCBs in a standard liquid (e.g., n-hexane) is fed into the standard container; the container is subjected to degassing treatment under vacuum for one hour so as to remove n-hexane; and the container is sealed and the temperature of the container is maintained at 100°C for one hour, thereby rendering the concentration of the PCBs uniform within the container.

Temperature-maintaining means 326 (e.g., a heater) is provided on the standard gas introduction tube 316 so as to maintain the temperature of the interior of the tube 316 at 150 ± 20°C, thereby preventing adhesion of an organic halogenated substance to the inner wall of the tube.

Preferably, the distance D between the standard gas introduction tube 316 and the standard container 312 is regulated such that the distance D is about 10 times the inner diameter (φ) of the standard gas introduction tube 316.

In the case where the distance D is short, when the valve 324 is opened, the heated gas enters the standard container. Therefore, in order to avoid such a problem, the distance D is regulated as described above.

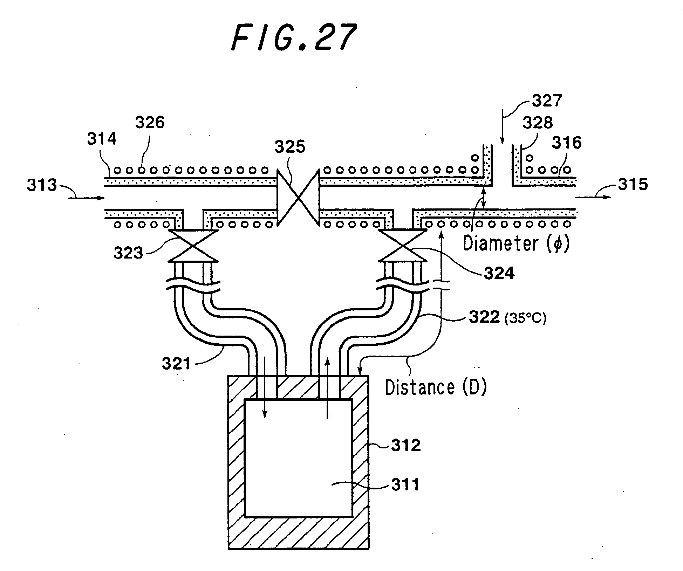

At present, the regulation limit of flue gas discharged from the aforementioned PCB treatment equipment is 0.15 mg/Nm³ (i.e., 15 ppb/V). Therefore, a standard gas containing PCB in an amount 1/5 the above value is employed for concentration correction in the mass spectrometer. As shown in FIG. 27, a dilution gas feed pipe 328 for feeding a dilution gas 327 (e.g., air or nitrogen) is provided on the standard gas introduction tube 316. The standard gas 315 is diluted with the dilution gas 327 so as to attain a predetermined concentration.

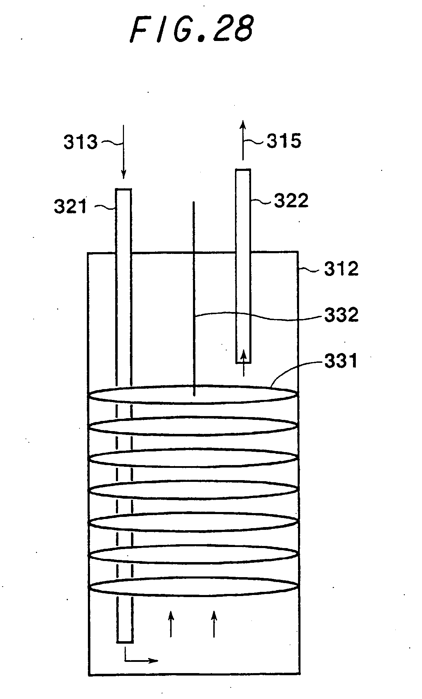

As shown in FIG. 28, the standard container 312 includes a plurality of disks 331, each having a plurality of pores; and the feed line 321 for feeding the purge gas 313 to the bottom of the container 312. The purge gas 313 is introduced to the bottom of the container 312, and then caused to pass through numerous pores of the disks 331, to thereby carry saturated PCBs to the outside of the container.

Thus, a PCB standard sample of uniform concentration can be introduced into the mass spectrometer.

In FIG. 28, reference numeral 332 denotes a thermometer.

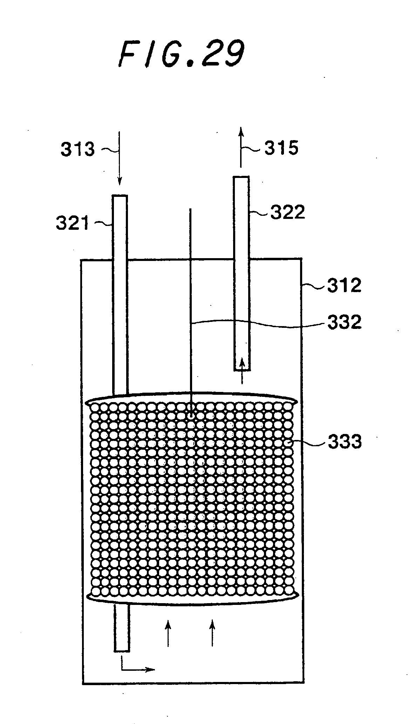

As shown in FIG. 29, instead of employing the disks 331, the standard container may be filled with glass fiber or beads 333.

The inner wall of the standard container 312 is covered with a coating layer formed of, for example, polytetrafluoroethylene or silicon oxide. Such a coating layer is provided in order to prevent PCBs from penetrating into the inner wall of the container.

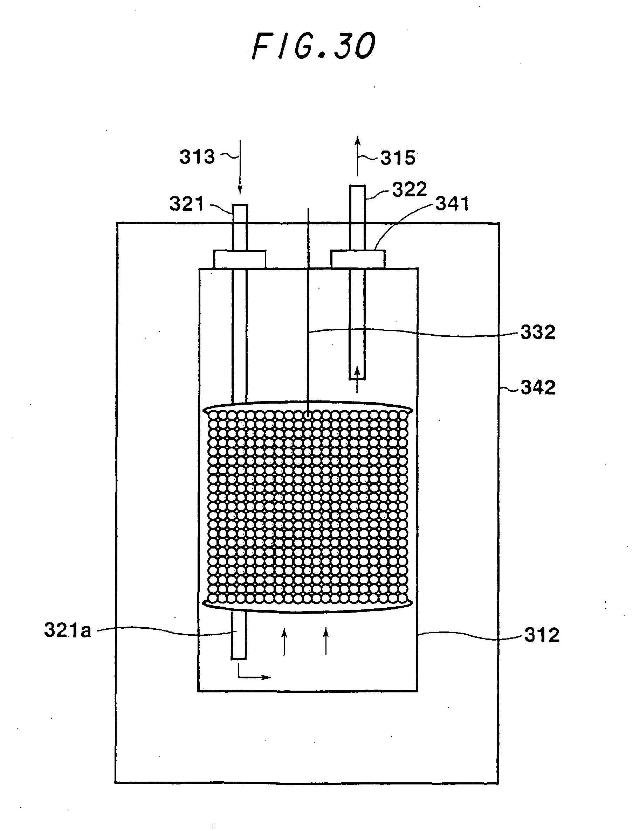

As shown in FIG. 30, the standard container 312 may be of detachable cartridge type having a detachable member 341.

Thus, detachment of the standard container 312 is readily attained, and any type of standard container can be provided.

As shown in FIG. 30, the detachable standard container 312 may be provided in a hermetic container 342. When the container 312 is provided in the hermetic container 342, leakage of PCBs to the outside can be prevented during the course of attachment/detachment of the container 312.

When a detection substance is fed into the standard container 312, and a sensor for detecting the detection substance is provided in the hermetic container 342, improper attachment of the container 312 can be discovered at an early stage.

The aforementioned detection substance is preferably hydrogen among other substances. When the purge gas 313 containing hydrogen in an amount of about some percent is fed into the container 312, and known detection means is provided on the inner wall of the hermetic container 342, leakage of the hydrogen can be detected quickly. As a result, improper attachment of the container 312 can be discovered through detection of the hydrogen (detection substance). The amount of hydrogen in the purge gas may be 100%. However, in consideration of leakage of hydrogen, the amount of hydrogen in the purge gas is preferably 4% (i.e., the lower explosive limit of hydrogen) or less.

When a line 300 for sampling an organic halogenated substance is provided in the hermetic container including the cartridge-type standard container 312, on-line measurement of the concentration of the organic halogenated substance can be performed by means of the measuring system 61 including the measuring apparatus 50 according to any embodiments.

In the case where the aforementioned halogenated substance concentration correction apparatus 310 is provided, when the concentration of PCBs in PCB treatment equipment is continuously measured, even if variation in measurement conditions arises in the time-of-flight mass spectrometer 60, such variation can be corrected quickly.

An internal standard gas 35 of predetermined concentration for monitoring (e.g., monochlorobenzene) is fed to the sample introduction tube 51 and to the purge gas feed tube 314. Variation in measurement conditions can be confirmed by monitoring the concentration of the gas 35.

In general, the concentration of PCBs as measured by means of the aforementioned apparatus is nearly equal to zero. Therefore, difficulty is encountered in determining whether the thus-measured PCB concentration is actually zero or the PCB concentration is inaccurately determined to be zero as a result of anomalous operation of the measuring apparatus or stuffing of pipes. However, when the aforementioned monitoring gas is fed, if the intensity of the peak corresponding to the thus-fed monitoring gas varies-although the intensity of the peak is generally found to be constant-anomalous operation of the measuring apparatus or stuffing of pipes can be discovered quickly.

The concentration of the standard gas 315 can be corrected by confirming that the ratio between the intensity of the peak corresponding to the monitoring gas 35 and that of the peak corresponding to the standard gas 315 is constant.

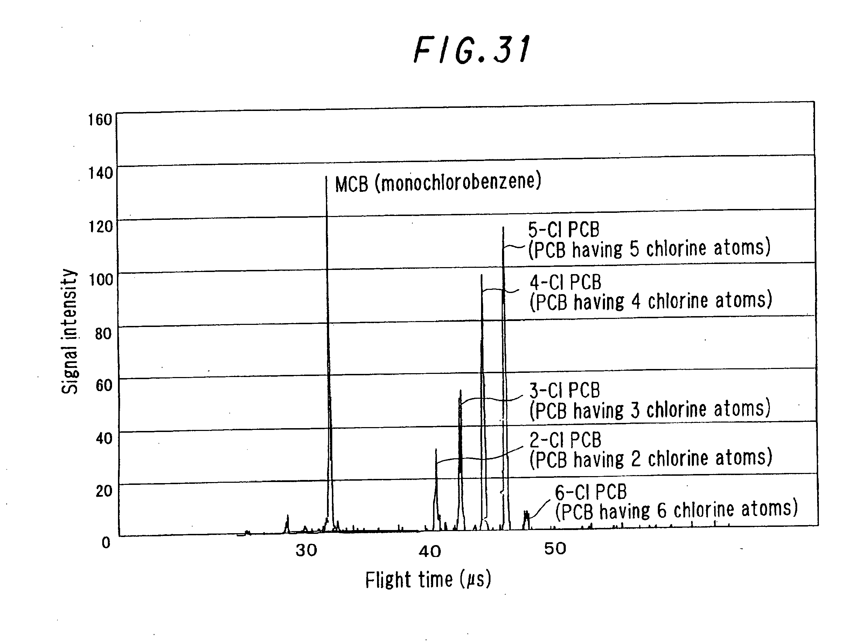

FIG. 31 is a chart showing results of measurement of the standard gas and the monitoring gas.

In the case where the concentration of PCBs is measured by means of the aforementioned measuring apparatus at predetermined intervals, when the gas for concentration correction is fed from the correction apparatus into the measuring apparatus after elapse of a predetermined period (e.g., one week or 10 days), the measuring apparatus can be operated properly.

### Industrial Applicability

As described above, the present invention provides an apparatus for detecting an organic halogenated substance contained in a gas. The detection apparatus includes sample introduction means for continuously introducing a collected sample into a vacuum chamber; laser irradiation means for irradiating the thus-introduced sample with a laser beam to thereby ionize the sample; a convergence section for converging molecules that have been ionized through laser irradiation; an ion trap for selectively trapping the thus-converged molecules; and a time-of-flight mass spectrometer incorporating an ion detector for detecting ions which are emitted at predetermined intervals. The detection apparatus enables quick analysis of organic halogenated substances such as PCBs and dioxins.

## Claims

1. An apparatus (50) for detecting an organic trace component comprising a polychlorinated biphenyl having 1 to 3 chlorine atoms, the apparatus comprising:
sample introduction means (54) for continuously introducing a collected sample (51) into a vacuum chamber (52);
laser irradiation means (66) for irradiating the thus-introduced sample with a laser beam (55) to thereby ionize the sample;
a convergence section (56) for converging molecules that have been ionized through laser irradiation;
an ion trap (57) for selectively trapping the thus-converged molecules; and
a time-of-flight mass spectrometer (60) incorporating an ion detector (59) for detecting ions which are emitted at predetermined intervals; **characterized in that**
the laser beam (55) radiated from the laser irradiation means (66) has a wavelength of 250 to 280 nm,
a pulse width of 100 to 500 picoseconds, and
an energy density of 0.01 to 0.1 GW/cm².

2. An apparatus (50) for detecting an organic trace component according to claim 1,
wherein the sample introduction means (54) is a capillary column, and the tip of the capillary column projects into the convergence section (56).

3. An apparatus (50) for detecting an organic trace component according to claim 1,
wherein the capillary column (54) is formed of quartz or stainless steel.

4. An apparatus (50) for detecting an organic trace component according to claim 1,
wherein the laser beam (55) radiated from the laser irradiation means (66) has a pulse frequency of at least 1 MHz.

5. An apparatus (50) for detecting an organic trace component according to claim 1,
wherein the organic trace component is a PCB contained in a gas in treatment equipment where PCB decomposition treatment has been performed.

6. An apparatus (50) for detecting an organic trace component according to claim 1,
wherein the organic trace component is a PCB contained in a flue gas or waste liquid discharged from treatment equipment where PCB decomposition treatment has been performed.

7. An apparatus (50) for detecting an organic trace component according to claim 1,
wherein the laser beam (55) has passed through a Raman cell (41).

8. An apparatus (50) for detecting an organic trace component according to claim 7,
wherein the Raman cell (41) contains hydrogen.

9. An apparatus (50) for detecting an organic trace component according to claim 1,
wherein the ion trap (57) comprises a first end cap electrode (81) having a small hole (81a) through which the ionized molecules enter, a second end cap electrode (82) having a small hole (82a) from which the trapped molecules are emitted, the first and second end cap electrode (81, 82) facing each other, and a high-frequency electrode (84) for applying a high-frequency voltage to the ion trap (57); the voltage of the first end cap electrode (81) is lower than that of the ion convergence section (56) for converging the ionized molecules, and the voltage of the second end cap electrode (82) is higher than that of the first end cap electrode (81); and the ionized molecules are trapped under application of the high-frequency voltage while the molecules within the ion trap (57) are selectively decelerated.

10. An apparatus (50) for detecting an organic trace component according to claim 9,
wherein an inert gas is caused to flow within the ion trap (57).

11. An apparatus (50) for detecting an organic trace component according to claim 9,
wherein an ionization zone (90) has a vacuum of 1.333 × 10⁻¹ Pa (1 × 10⁻³ torr), the ion convergence section (56) and the ion trap (57) have a vacuum of 1.333 × 10⁻³ Pa (1 × 10⁻⁵ torr), and the time-of-flight mass spectrometer (60) has a vacuum of 1.333 × 10⁻⁵ Pa (1 × 10⁻⁷ torr).

12. An apparatus (50) for detecting an organic trace component according to claim 1,
wherein the laser beam (55) radiated from the laser irradiation means (66) is repeatedly reflected such that the thus-reflected laser beams do not overlap one another within the ionization zone (90).

13. An apparatus (50) for detecting an organic trace component according to claim 12,
wherein the laser beam (55) radiated from the laser irradiation means (66) is repeatedly reflected by use of facing prisms (71, 72) such that the thus-reflected laser beams do not pass through the same path.

14. Use of an organic halogenated substance concentration correction apparatus (310) for correcting the organic trace component detection apparatus (50) according to claim 1,
wherein said organic halogenated substance concentration correction apparatus comprises a standard container (312) containing an organic halogenated substance (311) of predetermined concentration; and
a standard gas introduction tube (316) for feeding into the standard container a purge gas (313) for purging the organic halogenated substance, to thereby introduce into a mass spectrometer (50) the organic halogenated substance (311) accompanied by the purge gas (313).

15. Use of an organic halogenated substance concentration correction apparatus (310) according to claim 14,
wherein said organic halogenated substance concentration correction apparatus further comprises temperature-maintaining means for maintaining the temperature of the standard container (312) at a temperature 5 to 100 degrees higher than the temperature of an atmosphere surrounding the container.

16. Use of an organic halogenated substance concentration correction apparatus (310) according to claim 14,
wherein said organic halogenated substance concentration correction apparatus further comprises temperature-maintaining means for maintaining the temperature of the standard gas introduction means at 150°C or higher.

17. Use of an organic halogenated substance concentration correction apparatus (310) according to claim 14,
wherein in said organic halogenated substance concentration correction apparatus a disk (331) having a plurality of pores is provided in the standard container (312).

18. Use of an organic halogenated substance concentration correction apparatus (310) according to claim 14,
wherein the standard container (312) is filled with glass fiber or beads (333).

19. Use of an organic halogenated substance concentration correction apparatus (310) according to claim 17,
wherein a feed tube (321) for feeding a purge gas (313) is provided at the bottom of the standard container (312) such that the outlet of the feed tube faces the bottom, and the fed purge gas is discharged from the upper portion of the container.

20. Use of an organic halogenated substance concentration correction apparatus (310) according to claim 14,
wherein the inner wall of the standard container (312) is covered with a coating layer formed of polytetrafluoroethylene or silicon oxide.

21. Use of an organic halogenated substance concentration correction apparatus (310) according to claim 14,
wherein the standard container (312) is removably provided.

22. Use of an organic halogenated substance concentration correction apparatus (310) according to claim 21,
wherein the removable standard container (312) is provided in a hermetic container (342).

23. Use of an organic halogenated substance concentration correction apparatus (310) according to claim 22,
wherein a detection substance is fed into the standard container, and a sensor for detecting the detection substance is provided in the hermetic container (342).

24. Use of an organic halogenated substance concentration correction apparatus (310) according to claim 23,
wherein the detection substance is hydrogen.

25. Use of an organic halogenated substance concentration correction apparatus (310) according to claim 14,
wherein the sample contains PCBs.

26. Use of an organic halogenated substance concentration correction apparatus (310) according to claim 14, wherein said organic halogenated substance concentration correction apparatus corrects at predetermined intervals the concentration of the organic halogenated substance that is detected by said organic trace component detection apparatus (50).

27. Use of an organic halogenated substance concentration correction apparatus (310) according to claim 26,
wherein the sample contains PCBs, and the organic halogenated substance is a PCB.

## Patentansprüche

1. Vorrichtung (50) zum Detektieren einer organischen Spurenkomponente, die ein polychloriertes Biphenyl, welches 1 bis 3 Chloratome aufweist, umfasst, wobei die Vorrichtung umfasst:
Mittel zum Einführen einer Probe (54) zum kontinuierlichen Einführen einer genommenen Probe (51) in eine Vakuumkammer (52);
Mittel zur Laserbestrahlung (66) zum Bestrahlen der somit eingeführten Probe mit einem Laserstrahl (55), um hierdurch die Probe zu ionisieren;
einen Konvergenzbereich (56) zum Konvergieren von Molekülen, die durch Laserbestrahlung ionisiert worden sind;
eine Ionenfalle (57) zum selektiven Einfangen der somit konvergierten Moleküle; und
ein Flugzeit-Massenspektrometer (60), in das ein Ionendetektor (59) zum Detektieren von Ionen, die in vorbestimmten Intervallen emittiert werden, eingebaut ist; **dadurch gekennzeichnet, dass**
der Laserstrahl (55), der von dem Mittel zur Laserbestrahlung (66) ausgestrahlt wird, eine Wellenlänge von 250 bis 280 nm, eine Pulsdauer von 100 bis 500 Pikosekunden und eine Energiedichte von 0,01 bis 0,1 GW/cm² aufweist.

2. Vorrichtung (50) zum Detektieren einer organischen Spurenkomponente gemäß Anspruch 1,
worin das Mittel zum Einführen einer Probe (54) eine Kapillarsäule ist und die Spitze der Kapillarsäule in den Konvergenzbereich (56) vorstößt.

3. Vorrichtung (50) zum Detektieren einer organischen Spurenkomponente gemäß Anspruch 1, worin die Kapillarsäure (54) aus Quarz oder Edelstahl gebildet ist.

4. Vorrichtung (50) zum Detektieren einer organischen Spurenkomponente gemäß Anspruch 1, worin der Laserstrahl (55), der von dem Mittel zur Laserbestrahlung (66) ausgestrahlt wird, eine Pulsfrequenz von mindestens 1 MHz aufweist.

5. Vorrichtung (50) zum Detektieren einer organischen Spurenkomponente gemäß Anspruch 1, worin die organische Spurenkomponente ein PCB ist, das in einem Gas in einer Behandlungsausrüstung enthalten ist, worin eine PCB-Zersetzungsbehandlung durchgeführt worden ist.

6. Vorrichtung (50) zum Detektieren einer organischen Spurenkomponente gemäß Anspruch 1, worin die organische Spurenkomponente ein PCB ist, das in einem Abgas oder einer Abfallflüssigkeit enthalten ist, das/die aus einer Behandlungsausrüstung ausgestoßen wird, worin eine PBC-Zersetzungsbehandlung durchgeführt worden ist.

7. Vorrichtung (50) zum Detektieren einer organischen Spurenkomponente gemäß Anspruch 1, worin der Laserstrahl (55) durch eine Raman-Zelle (41) geführt wurde.

8. Vorrichtung (50) zum Detektieren einer organischen Spurenkomponente gemäß Anspruch 7, worin die Raman-Zelle (41) Wasserstoff enthält.

9. Vorrichtung (50) zum Detektieren einer organischen Spurenkomponente gemäß Anspruch 1,
worin die Ionenfalle (57) eine erste Endkappenelektrode (81), die ein kleines Loch (81a) aufweist, durch das die ionisierten Moleküle eindringen, eine zweite Endkappenelektrode (82), die ein kleines Loch (82a) aufweist, aus dem die eingefangenen Moleküle emittiert werden, wobei die erste und die zweite Endkappenelektrode (81, 82) gegenüberliegend angeordnet sind, und eine Hochfrequenzelektrode (84) zum Anlegen einer Hochfrequenzspannung an die Ionenfalle (57) umfasst; wobei die Spannung der ersten Endkappenelektrode (81) niedriger ist als die des Ionenkonvergenzbereichs (56) zum Konvergieren der ionisierten Moleküle, und wobei die Spannung der zweiten Endkappenelektrode (82) höher ist als die der ersten Endkappenelektrode (81); und worin die ionisierten Moleküle unter Anlegung der Hochfrequenzspannung gefangen werden, während die Moleküle innerhalb der Ionenfalle (57) selektiv abgebremst werden.

10. Vorrichtung (50) zum Detektieren einer organischen Spurenkomponente gemäß Anspruch 9, worin ein Inertgas innerhalb der Ionenfalle (57) fließengelassen wird.

11. Vorrichtung (50) zum Detektieren einer organischen Spurenkomponente gemäß Anspruch 9, worin die Ionisierungszone (90) ein Vakuum von 1,333 x 10⁻¹ Pa (1x 10⁻³ Torr) aufweist, der Ionenkonvergenzbereich (56) und die Ionenfalle (67) ein Vakuum von 1,333 x 10⁻³ Pa (1 x 10⁻⁵ Torr) aufweisen und das Flugzeit-Massenspektrometer (60) ein Vakuum von 1,333 x 10⁻⁵ Pa (1 x 10⁻⁷ Torr) aufweist.

12. Vorrichtung (50) zum Detektieren einer organischen Spurenkomponente gemäß Anspruch 1, worin der Lasterstrahl (55), der von dem Mittel zur Laserbestrahlung (66) ausgestrahlt wird, wiederholt so reflektiert wird, dass die somit reflektieren Laserstrahlen sich innerhalb der Ionisierungszone (90) nicht miteinander überlappen.

13. Vorrichtung (50) zum Detektieren einer organischen Spurenkomponente gemäß Anspruch 12, worin der Laserstrahl (55), der von dem Mittel zur Laserbestrahlung (66) ausgestrahlt wird, wiederholt unter Verwendung von sich gegenüberliegenden Prismen (71, 72) so reflektiert wird, dass die somit reflektierten Lasterstrahlen nicht durch den gleichen Weg laufen.

14. Verwendung einer Vorrichtung zur Korrektur der Konzentration einer organischen halogenierten Substanz (310) zum Korrigieren der Vorrichtung zur Detektion einer organischen Spurenkomponente (50) gemäß Anspruch 1,
worin die Vorrichtung zur Korrektur der Konzentration einer organischen halogenierten Substanz einen Standardcontainer (312), der eine organische halogenierte Substanz (311) mit vorbestimmter Konzentration enthält; und
eine Standardgas-Einführröhre (316) zum Zuführen eines Spülgases (313) in den Standardcontainer zum Spülen der organischen halogenierten Substanz umfasst, um hierdurch die organische halogenierte Substanz (311), die von dem Spülgas (313) begleitet wird, in das Massenspektrometer (50) einzuführen.

15. Verwendung einer Vorrichtung zur Korrektur der Konzentration einer organischen halogenierten Substanz (310) gemäß Anspruch 14, worin die Vorrichtung zur Korrektur der Konzentration einer organischen halogenierten Substanz ferner ein Mittel zur Beibehaltung der Temperatur zum Beibehaltung der Temperatur des Standardcontainers (312) bei einer Temperatur umfasst, die 5 bis 100 Grad höher ist als die Temperatur der Atmosphäre, die den Container umgibt.

16. Verwendung einer Vorrichtung zur Korrektur der Konzentration einer organischen halogenierten Substanz (310) gemäß Anspruch 14, worin die Vorrichtung zur Korrektur der Konzentration einer organischen halogenierten Substanz ferner ein Mittel zur Beibehaltung der Temperatur zum Beibehalten der Temperatur des Standardgas-Einführmittels bei 150°C oder höher umfasst.

17. Verwendung einer Vorrichtung zur Korrektur der Konzentration einer organischen halogenierten Substanz (310) gemäß Anspruch 14, worin in der Vorrichtung zur Korrektur der Konzentration einer organischen halogenierten Substanz eine Scheibe (331) mit einer Vielzahl von Poren in dem Standardcontainer (312) vorgesehen ist.

18. Verwendung einer Vorrichtung zur Korrektur der Konzentration einer organischen halogenierten Substanz (310) gemäß Anspruch 14, worin der Standardcontainer (312) mit Glasfasern oder Kügelchen (333) gefüllt ist.

19. Verwendung einer Vorrichtung zur Korrektur der Konzentration einer organischen halogenierten Substanz (310) gemäß Anspruch 17, worin ein Zuführrohr (321) zum Zuführen eines Spülgases (313) am Boden des Standardcontainers (312) so vorgesehen ist, dass der Auslass des Zuführrohrs dem Boden zugewandt ist und das zugeführte Spülgas vom oberen Bereich des Containers ausgeführt wird.

20. Verwendung einer Vorrichtung zur Korrektur der Konzentration einer organischen halogenierten Substanz (310) gemäß Anspruch 14, worin die innere Wand des Standardcontainers (312) mit einer Beschichtungsschicht bedeckt ist, die aus Polytetrafluorethylen oder Siliciumoxid gebildet ist.

21. Verwendung einer Vorrichtung zur Korrektur der Konzentration einer organischen halogenierten Substanz (310) gemäß Anspruch 14, worin der Standardcontainer (312) entfernbar vorgesehen ist.

22. Verwendung einer Vorrichtung zur Korrektur der Konzentration einer organischen halogenierten Substanz (310) gemäß Anspruch 21, worin der entfernbare Standardcontainer (312) in einem hermetischen Container (342) vorgesehen ist.

23. Verwendung einer Vorrichtung zur Korrektur der Konzentration einer organischen halogenierten Substanz (310) gemäß Anspruch 22, worin eine Detektionssubstanz zu dem Standardcontainer zugeführt wird und ein Sensor zum Detektieren der Detektionssubstanz in dem hermetischen Container (342) vorgesehen ist.

24. Verwendung einer Vorrichtung zur Korrektur der Konzentration einer organischen halogenierten Substanz (310) gemäß Anspruch 23, worin die Detektionssubstanz Wasserstoff ist.

25. Verwendung einer Vorrichtung zur Korrektur der Konzentration einer organischen halogenierten Substanz (310) gemäß Anspruch 14, worin die Probe PCBs enthält.

26. Verwendung einer Vorrichtung zur Korrektur der Konzentration einer organischen halogenierten Substanz (310) gemäß Anspruch 14, worin die Vorrichtung zur Korrektur der Konzentration einer organischen halogenierten Substanz in vorbestimmten Intervallen die Konzentration der organischen halogenierten Substanz korrigiert, die von der Vorrichtung zur Detektion einer organischen Spurenkomponente (50) detektiert wird.

27. Verwendung einer Vorrichtung zur Korrektur der Konzentration einer organischen halogenierten Substanz (310) gemäß Anspruch 26, worin die Probe PCBs enthält und die organische halogenierte Substanz ein PCB ist.

## Revendications

1. Appareil (50) permettant de détecter un composant organique à l'état de trace comprenant un biphényle polychloré contenant 1 à 3 atomes de chlore, l'appareil comprenant :
un moyen d'introduction d'échantillon (54) servant à introduire en continu dans une chambre à vide (52) un échantillon collecté (51);
un moyen d'irradiation par laser (66) servant à irradier l'échantillon ainsi introduit avec un faisceau laser (55) pour ainsi ioniser l'échantillon ;
une section de convergence (56) servant à faire converger les molécules qui ont été ionisées par l'irradiation laser ;
un piège à ions (57) servant à piéger sélectivement les molécules qui ont ainsi convergé ; et
un spectromètre de masse à temps de vol (60) incorporant un détecteur d'ions (59) servant à détecter des ions qui sont émis à des intervalles prédéterminés ; **caractérisé en ce que**
le faisceau laser (55) produit par le moyen d'irradiation par laser (66) possède une longueur d'onde allant de 250 nm à 280 nm,
une largeur d'impulsion allant de 100 picosecondes à 500 picosecondes, et
une densité d'énergie allant de 0,01 GW/cm² à 0,1 GW/cm².

2. Appareil (50) permettant de détecter un composant organique à l'état de trace selon la revendication 1,
dans lequel le moyen d'introduction d'échantillon (54) est une colonne capillaire, et l'extrémité de la colonne capillaire se projette dans la section de convergence (56).

3. Appareil (50) permettant de détecter un composant organique à l'état de trace selon la revendication 1,
dans lequel la colonne capillaire (54) est formée de quartz ou d'acier inoxydable.

4. Appareil (50) permettant de détecter un composant organique à l'état de trace selon la revendication 1,
dans lequel le faisceau laser (55) produit par le moyen d'irradiation par laser (66) présente une fréquence d'impulsion d'au moins 1 MHz.

5. Appareil (50) permettant de détecter un composant organique à l'état de trace selon la revendication 1,
dans lequel le composant organique à l'état de trace est un PCB contenu dans un gaz dans un équipement de traitement où un traitement de décomposition de PCB a été effectué.

6. Appareil (50) permettant de détecter un composant organique à l'état de trace selon la revendication 1,
dans lequel le composant organique à l'état de trace est un PCB contenu dans un gaz d'évacuation ou un déchet liquide déchargé d'un équipement de traitement où un traitement de décomposition de PCB a été effectué.

7. Appareil (50) permettant de détecter un composant organique à l'état de trace selon la revendication 1,
dans lequel le faisceau laser (55) a traversé une cellule Raman (41).

8. Appareil (50) permettant de détecter un composant organique à l'état de trace selon la revendication 7,
dans lequel la cellule Raman (41) contient de l'hydrogène.

9. Appareil (50) permettant de détecter un composant organique à l'état de trace selon la revendication 1,
dans lequel le piège à ions (57) comprend une première électrode d'extrémité (81) présentant un petit trou (81a) à travers lequel les molécules ionisées entrent, une seconde électrode d'extrémité (82) présentant un petit trou (82a) à partir duquel les molécules piégées sont émises, la première et la seconde électrode d'extrémité (81, 82) étant l'une en face de l'autre, et une électrode à haute fréquence (84) servant à appliquer une tension à haute fréquence au piège à ions (57) ; la tension de la première électrode d'extrémité (81) est inférieure à celle de la section de convergence d'ions (56) pour faire converger les molécules ionisées, et la tension de la seconde électrode d'extrémité (82) est supérieure à celle de la première électrode d'extrémité (81) ; et les molécules ionisées sont piégées lors de l'application de la tension à haute fréquence pendant que les molécules se trouvant dans le piège à ions (57) sont sélectivement décélérées.

10. Appareil (50) permettant de détecter un composant organique à l'état de trace selon la revendication 9,
dans lequel un gaz inerte est amené à s'écouler dans le piège à ions (57).

11. Appareil (50) permettant de détecter un composant organique à l'état de trace selon la revendication 9,
dans lequel une zone d'ionisation (90) présente un vide de 1,333 x 10⁻¹ Pa (1 x 10⁻³ torr), la section de convergence d'ions (56) et le piège à ions (57) présentent un vide de 1,333 x 10⁻³ Pa (1 x 10⁻⁵ torr), et le spectromètre de masse à temps de vol (60) présente un vide de 1,333 x 10⁻⁵ Pa (1 x 10⁻⁷ torr).

12. Appareil (50) permettant de détecter un composant organique à l'état de trace selon la revendication 1,
dans lequel le faisceau laser (55) produit par le moyen d'irradiation par laser (66) est réfléchi plusieurs fois de telle sorte que les faisceaux lasers ainsi réfléchis ne se chevauchent par les uns par rapport aux autres dans la zone d'ionisation (90).

13. Appareil (50) permettant de détecter un composant organique à l'état de trace selon la revendication 12,
dans lequel le faisceau laser (55) produit par le moyen d'irradiation par laser (66) est réfléchi plusieurs fois par l'utilisation de prismes face-à-face (71, 72) de telle sorte que les faisceaux lasers ainsi réfléchis ne passent pas par le même trajet.

14. Utilisation d'un appareil de correction de la concentration d'une substance organique halogénée (310) pour corriger l'appareil de détection d'un composant organique à l'état de trace (50) selon la revendication 1,
dans lequel ledit appareil de correction de la concentration d'une substance organique halogénée comprend un récipient standard (312) contenant une substance organique halogénée (311) à une concentration prédéterminée ; et
un tube standard d'introduction de gaz (316) servant à introduire dans le récipient standard un gaz de purge (313) pour purger la substance organique halogénée, pour ainsi introduire dans un spectromètre de masse (50) la substance organique halogénée (311) accompagnée par le gaz de purge (313).

15. Utilisation d'un appareil de correction de la concentration d'une substance organique halogénée (310) selon la revendication 14,
dans laquelle ledit appareil de correction de la concentration d'une substance organique halogénée comprend en outre un moyen de maintien de la température servant à maintenir la température du récipient standard (312) à une température supérieure de 5 à 100 degrés à la température d'une atmosphère entourant le récipient.

16. Utilisation d'un appareil de correction de la concentration d'une substance organique halogénée (310) selon la revendication 14,
dans laquelle ledit appareil de correction de la concentration d'une substance organique halogénée comprend en outre un moyen de maintien de la température servant à maintenir la température du moyen standard d'introduction de gaz à une température supérieure ou égale à 150 °C.

17. Utilisation d'un appareil de correction de la concentration d'une substance organique halogénée (310) selon la revendication 14,
dans laquelle, dans ledit appareil de correction de la concentration d'une substance organique halogénée, un disque (331) présentant une pluralité de pores est placé dans le récipient standard (312).

18. Utilisation d'un appareil de correction de la concentration d'une substance organique halogénée (310) selon la revendication 14,
dans laquelle le récipient standard (312) est rempli avec des fibres ou des billes de verre (333).

19. Utilisation d'un appareil de correction de la concentration d'une substance organique halogénée (310) selon la revendication 17,
dans laquelle un tube d'alimentation (321) servant à introduire un gaz de purge (313) est placé sur la partie inférieure du récipient standard (312), de telle sorte que l'orifice de sortie du tube d'alimentation se trouve en face de la partie inférieure et que le gaz de purge introduit soit déchargé par la partie supérieure du récipient.

20. Utilisation d'un appareil de correction de la concentration d'une substance organique halogénée (310) selon la revendication 14,
dans laquelle la paroi interne du récipient standard (312) est recouverte avec une couche de revêtement formée de polytétrafluoroéthylène ou d'oxyde de silicium.

21. Utilisation d'un appareil de correction de la concentration d'une substance organique halogénée (310) selon la revendication 14,
dans laquelle le récipient standard (312) est amovible.

22. Utilisation d'un appareil de correction de la concentration d'une substance organique halogénée (310) selon la revendication 21,
dans laquelle le récipient standard amovible (312) est fourni dans un récipient hermétique (342).

23. Utilisation d'un appareil de correction de la concentration d'une substance organique halogénée (310) selon la revendication 22,
dans laquelle une substance de détection est introduite dans le récipient standard et un capteur servant à détecter la substance de détection est placé dans le récipient hermétique (342).

24. Utilisation d'un appareil de correction de la concentration d'une substance organique halogénée (310) selon la revendication 23,
dans laquelle la substance de détection est l'hydrogène.

25. Utilisation d'un appareil de correction de la concentration d'une substance organique halogénée (310) selon la revendication 14,
dans laquelle l'échantillon contient des PCB.

26. Utilisation d'un appareil de correction de la concentration d'une substance organique halogénée (310) selon la revendication 14,
dans laquelle ledit appareil de correction de la concentration d'une substance organique halogénée corrige à des intervalles prédéterminés la concentration de la substance organique halogénée qui est détectée par ledit appareil de détection d'un composant organique à l'état de trace (50).

27. Utilisation d'un appareil de correction de la concentration d'une substance organique halogénée (310) selon la revendication 26,
dans laquelle l'échantillon contient des PCB et la substance organique halogénée est un PCB.
